# EUROPEAN PATENT SPECIFICATION

(11) **EP 3 021 689 B1**
(45) Date of publication and mention of the grant of the patent: **24.03.2021**
(21) Application number: 14744489.7
(22) Date of filing: 15.07.2014
(51) Int. Cl.: A23L 27/30, C12P 21/00, C12N 15/00, C12P 19/56, A23G 3/36, A23G 4/06, A23G 9/32, C12N 15/52, C12P 5/00

(54) **DITERPENE PRODUCTION**
HERSTELLUNG VON DITERPEN
PRODUCTION DE DITERPÈNE

(30) Priority: 15.07.2013 US 201313942491
(43) Date of publication of application: 25.05.2016
(73) Proprietor: DSM IP Assets B.V., 6411 TE Heerlen (NL)
(72) Inventor: HOEVEN, VAN DER, Robertus, Antonius, Mijndert, 6100 AA Echt (NL); GALAEV, Igor, 6100 AA Echt (NL); BOER, Viktor Marius, 6100 AA Echt (NL)
(74) Representative: DSM Intellectual Property
(86) International application number: PCT/EP2014/065179
(87) International publication number: WO 2015/007748

(56) References cited:
- WO-A1-2011/153378
- WO-A1-2012/075030
- WO-A1-2013/096420
- WO-A1-2013/110673
- WO-A1-2013/176738
- WO-A1-2014/122328
- WO-A2-2013/022989
- WO-A2-2014/122227
- TANIA V HUMPHREY ET AL: "Spatial Organisation of Four Enzymes from Stevia rebaudiana that are Involved in Steviol Glycoside Synthesis", PLANT MOLECULAR BIOLOGY, KLUWER ACADEMIC PUBLISHERS, DORDRECHT, NL, vol. 61, no. 1-2, 1 May 2006 (2006-05-01), pages 47-62, XP019405427, ISSN: 1573-5028, DOI: 10.1007/S11103-005-5966-9
- BRANDLE ET AL: "Steviol glycoside biosynthesis", PHYTOCHEMISTRY, PERGAMON PRESS, GB, vol. 68, no. 14, 1 July 2007 (2007-07-01), pages 1855-1863, XP022145443, ISSN: 0031-9422, DOI: 10.1016/J.PHYTOCHEM.2007.02.010

## Description

### Field

The present invention relates to a recombinant microorganism capable of producing a diterpene and/or a glycosylated diterpene and to a process for the production of a diterpene and/or a glycosylated diterpene by use of such a cell. The invention further relates to a fermentation broth comprising a diterpene and/or glycosylated diterpene obtainable by such a process. The invention also relates to a method for converting a first glycosylated diterpene into a second glycosylated diterpene

### Background

The worldwide demand for high potency sweeteners is increasing and, with blending of different artificial sweeteners, becoming a standard practice. However, the demand for alternatives is expected to increase. The leaves of the perennial herb, *Stevia rebaudiana* Bert., accumulate quantities of intensely sweet compounds known as steviol glycosides. Whilst the biological function of these compounds is unclear, they have commercial significance as alternative high potency sweeteners, with the added advantage that *Stevia* sweeteners are natural plant products.

These sweet steviol glycosides have functional and sensory properties that appear to be superior to those of many high potency sweeteners. In addition, studies suggest that stevioside can reduce blood glucose levels in Type II diabetics and can reduce blood pressure in mildly hypertensive patients.

Steviol glycosides accumulate in Stevia leaves where they may comprise from 10 to 20% of the leaf dry weight. Stevioside and rebaudioside A are both heat and pH stable and suitable for use in carbonated beverages and many other foods. Stevioside is between 110 and 270 times sweeter than sucrose, rebaudioside A between 150 and 320 times sweeter than sucrose. In addition, rebaudioside D is also a high-potency diterpene glycoside sweetener which accumulates in Stevia leaves. It may be about 200 times sweeter than sucrose. Rebaudioside M s also a high-potency diterpene glycoside sweetener present in trace amount in certain stevia variety leaves but has been suggested to have best taste profile.

Currently, steviol glycosides are extracted from the *Stevia* plant. In *Stevia,* (-)-kaurenoic acid, an intermediate in gibberellic acid (GA) biosynthesis, is converted into the tetracyclic dipterepene steviol, which then proceeds through a multi-step glucosylation pathway to form the various steviol glycosides. However, yields may be variable and affected by agriculture and environmental conditions. Also, Stevia cultivation requires substantial land area, a long time prior to harvest, intensive labour and additional costs for the extraction and purification of the glycosides.

New, more standardized, clean single composition, no after-taste, sources of glycosides, such as rebaudioside M, are required to meet growing commercial demand for high potency, natural sweeteners.

### Summary

Development of fermentation technologies for production of high-value steviol glycosides that may be lower-cost, more abundant, and currently perceived as trace byproducts is desired.

There are more than 30 different steviol glycosides found within the stevia leaf, including Reb A, and next-generation sweeteners such as Reb D and Reb M, which have superior taste profiles but which are found in much lower quantities within the stevia leaf. Because the most desirable steviol glycosides are rare within the stevia leaf, fermentation processes offer attractive commercial advantages for large-scale production. This invention illustratess a method for quickly and reliably produce a variety of next-generation stevia sweeteners in an on-demand fashion and that could be further tailored to changing consumer demands for stevia.

In Stevia, steviol is synthesized from GGPP, which is formed by the deoxyxylulose 5- phosphate pathway. The activity of two diterpene cyclases (-)-copalyl diphosphate synthase (CPS) and (-)-kaurene synthase (KS) results in the formation of (-)-Kaurene which is then oxidized in a three step reaction by (-)-kaurene oxidase (KO) to form (-)-kaurenoic acid.

In Stevia leaves, (-)-kaurenoic acid is then hydroxylated, by ent-kaurenoic acid 13-hydroxylase (KAH) to form steviol. Steviol is then glucosylated by a series of UDP-glucosyltransferases (UGTs).

The invention is set forward in the claims herein. Disclosed is a microorganism capable of producing a diterpene, such as steviol, or a glycosylated diterpene (i.e. a diterpene glycoside), such as steviolmonoside, steviolbioside, stevioside, rebaudioside A, rebaudioside B, rebaudioside C, rebaudioside D, rebaudioside E, rebaudioside F, rubusoside or dulcoside A.

The disclosure thus provides a recombinant microorganism comprising one or more nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity,
a polypeptide capable of catalyzing the addition of a glucose at the C-13 position of steviol to yield 13-*O*-steviolmonoside,
a polypeptide capable of catalyzing the addition of a glucose at the C-13 position of 13-*O*-steviolmonoside to yield steviolbioside or at the C-19 position of rebaudioside A to yield rebaudioside D,
a polypeptide capable of catalyzing the addition of a glucose at the C-19 position of steviolbioside to yield stevioside; and
a polypeptide capable of catalyzing addition of a glucose at the C-13 position of stevioside to yield rebaudioside A or at the C-19 position of rebaudioside D to yield rebaudioside M,
whereby expression of the nucleotide sequence(s) confer(s) on the microorganism the ability to produce at least rebaudioside M.

Typically, a recombinant microorganism disclosedwill comprise all of the above nucleotide sequences.

The disclosure also provides:
- a process for the preparation of rebaudioside M which comprises fermenting a recombinant microorganism disclosedin a suitable fermentation medium, and optionally recovering the diterpene or glycosylated diterpene;
- a fermentation broth comprising rebaudioside M obtainable by the process disclosed;
- rebaudioside M obtained by a process according to the disclosure or obtainable from a fermentation broth disclosed;
- a foodstuff, feed or beverage which comprises rebaudioside M according to the disclosure.

### Brief description of the drawings

Figure 1 sets out a schematic representation of the plasmid pUG7-EcoRV.
Figure 2 sets out a schematic representation of the method by which the ERG20, tHMG1 and BTS1 over-expression cassettes are designed (A) and integrated (B) into the yeast genome. (C) shows the final situation after removal of the KANMX marker by the Cre recombinase.
Figure 3 sets out a schematic representation of the ERG9 knock down construct. This consists of a 500 bp long 3' part of *ERG9,* 98 bp of the *TRP1* promoter, the *TRP1* open reading frame and terminator, followed by a 400 bp long downstream sequence of *ERG9.* Due to introduction of a Xbal site at the end of the *ERG9* open reading frame the last amino acid changes into Ser and the stop codon into Arg. A new stop codon is located in the *TPR1* promoter, resulting in an extension of 18 amino acids.
Figure 4 sets out a schematic representation of how UGT2 is integrated into the genome. A. different fragments used in transformation; B. situation after integration; C. situation after expression of Cre recombinase.
Figure 5 sets out a schematic representation of how the pathway from GGPP to RebA is integrated into the genome. A. different fragments used in transformation; B. situation after integration.

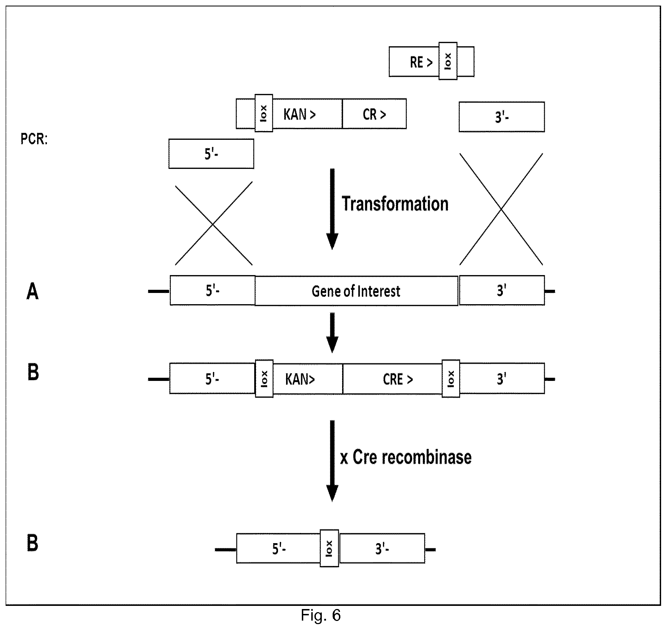

Figure 6 sets out a schematic representation of how specific gene deletions are constructed. A. genome in the parent strain; B. situation after integration; C. situation after out-recombination
Figure 7 sets out a schematic diagram of the potential pathways leading to biosynthesis of steviol glycosides.
Figure 8 sets out analytical HPLC chromatograms generated in the preparation of RebM. From the top: concentrate extraction; centrifugated feed (diluted concentrate); eluate after 1^{st} chrom run; eluate after 2^{nd} chrom run (pH8,5); and RebA standard.
Figure 9 sets out a schematic representation of the plasmid MB6969.
Figure 10 sets out a schematic representation of the plasmid MB6856.
Figure 11 sets out a schematic representation of the plasmid MB6857.
Figure 12 sets out a schematic representation of the plasmid MB6948.
Figure 13 sets out a schematic representation of the plasmid MB6958.
Figure 14 sets out a schematic representation of the plasmid MB7015.
Figure 15 sets out a schematic representation of the plasmid MB6986.
Figure 16 sets out a schematic representation of the plasmid MB7059.
Figure 17 sets out a schematic representation of the plasmid MB7100.
Figure 18 sets out a schematic representation of the plasmid MB6988.
Figure 19 sets out a schematic representation of the plasmid MB7044.
Figure 20 sets out a schematic representation of the plasmid MB7094.
Figure 21 sets out a schematic representation of the plasmid pRS417 Con5-3.

### Description of the sequence listing

A description of the sequences is set out in Table 1. Sequences described herein may be defined with reference to the sequence listing or with reference to the database accession numbers also set out in Table 1.

### Detailed description of the disclosure

The invention is set forward in the claims herein. Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

The invention relates to a recombinant microorganism that is capable of producing rebaudioside M (RebM). The structure of RebM is set out in Figure and in J Appl. Glycosci. 57, 199-209, 2010.

RebM is a glycosylated diterpene. For the purposes of this invention, a diterpene typically means an organic compound composed of four isoprene units. Such a compound may be derived from geranylgeranyl pyrophosphate. A glycosylated diterpene or diterpene glycoside is a diterpene in which a sugar is bound, typically to a non-carbohydrate moiety. Typically, in a diterpene glycoside, the sugar group may be bonded through its anomeric carbon to another group via a glycosidic bond.

Disclosed is a recombinant microorganism. The recombinant microorganism comprises one or more nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity.

For the purposes of this invention, a polypeptide having *ent*-copalyl pyrophosphate synthase (EC 5.5.1.13) is capable of catalyzing the chemical reation:

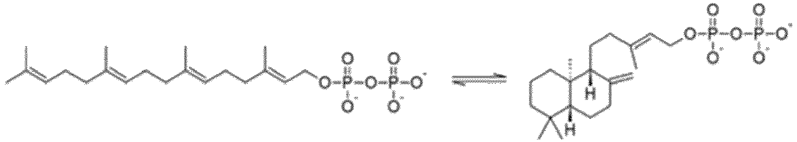

This enzyme has one substrate, geranylgeranyl pyrophosphate, and one product, *ent*-copalyl pyrophosphate. This enzyme participates in gibberellin biosynthesis. This enzyme belongs to the family of isomerase, specifically the class of intramolecular lyases. The systematic name of this enzyme class is *ent*-copalyl-diphosphate lyase (decyclizing). Other names in common use include having *ent*-copalyl pyrophosphate synthase, *ent*-kaurene synthase A, and *ent*-kaurene synthetase A.

For the purposes of this invention, a polypeptide having ent-kaurene synthase activity (EC 4.2.3.19) is a polypeptide that is capable of catalyzing the chemical reaction:

ent-copalyl diphosphate ⇄ent-kaurene + diphosphate

Hence, this enzyme has one substrate, ent-copalyl diphosphate, and two products, ent-kaurene and diphosphate.

This enzyme belongs to the family of lyases, specifically those carbon-oxygen lyases acting on phosphates. The systematic name of this enzyme class is ent-copalyl-diphosphate diphosphate-lyase (cyclizing, ent-kaurene-forming). Other names in common use include ent-kaurene synthase B, ent-kaurene synthetase B, ent-copalyl-diphosphate diphosphate-lyase, and (cyclizing). This enzyme participates in diterpenoid biosynthesis.

*ent*-copalyl diphosphate synthases may also have a distinct *ent*-kaurene synthase activity associated with the same protein molecule. The reaction catalyzed by *ent*-kaurene synthase is the next step in the biosynthetic pathway to gibberellins. The two types of enzymic activity are distinct, and site-directed mutagenesis to suppress the *ent*-kaurene synthase activity of the protein leads to build up of *ent*-copalyl pyrophosphate.

Accordingly, a single nucleotide sequence disclosed may encode a polypeptide having *ent*-copalyl pyrophosphate synthase activity and *ent*-kaurene synthase activity. Alternatively, the two activities may be encoded two distinct, separate nucleotide sequences.

For the purposes of this invention, a polypeptide having ent-kaurene oxidase activity (EC 1.14.13.78) is a polypeptide which is capable of catalysing three successive oxidations of the 4-methyl group of ent-kaurene to give kaurenoic acid. Such activity typically requires the presence of a cytochrome P450.

For the purposes of the invention, a polypeptide having kaurenoic acid 13-hydroxylase activity (EC 1.14.13) is one which is capable of catalyzing the formation of steviol (ent-kaur-16-en-13-ol-19-oic acid) using NADPH and O₂. Such activity may also be referred to as ent-ka 13-hydroxylase activity.

A recombinant microorganism disclosed also comprises nucleotide sequences encoding polypeptides having UDP-glucosyltransferase (UGT) activity, whereby expression of the nucleotide sequences confers on the microorganism the ability to produce at least rebaudioside M.

For the purposes of this invention, a polypeptide having UGT activity is one which has glycosyltransferase activity (EC 2.4), i.e. that can act as a catalyst for the transfer of a monosaccharide unit from an activated nucleotide sugar (also known as the "glycosyl donor") to a glycosyl acceptor molecule, usually an alcohol. The glycosyl donor for a UGT is typically the nucleotide sugar uridine diphosphate glucose (uracil-diphosphate glucose, UDP-glucose).

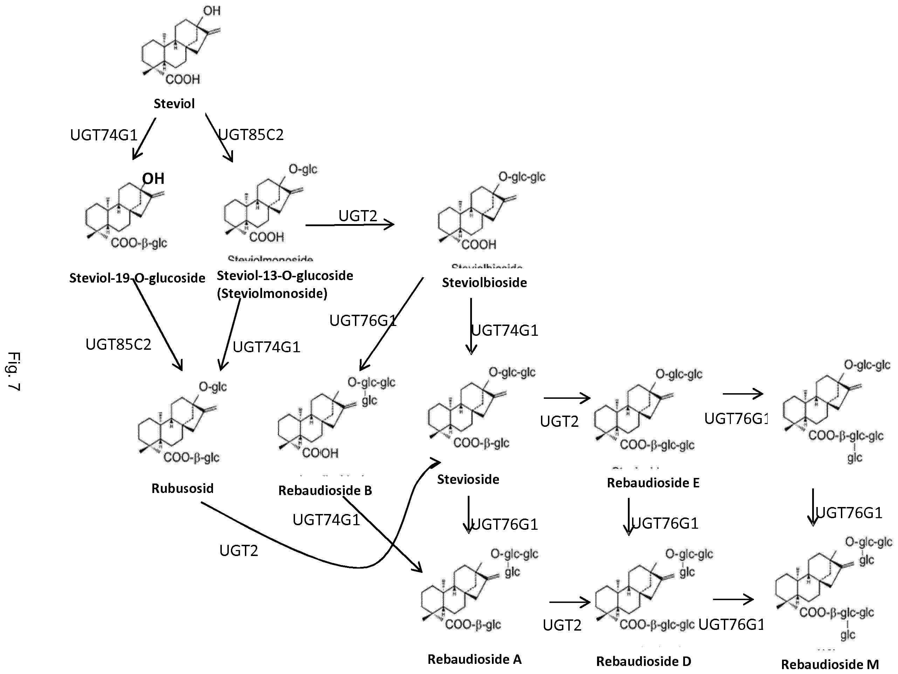

The UGTs used are selected so as to produce a desired diterpene glycoside, such as a steviol glycoside. Schematic diagrams of steviol glycoside formation are set out in Humphrey et al., Plant Molecular Biology (2006) 61: 47-62 and Mohamed et al., J. Plant Physiology 168 (2011) 1136-1141. In addition, Figure 7 sets out a schematic diagram of steviol glycoside formation.

The biosynthesis of rebaudioside A involves glucosylation of the aglycone steviol. Specifically, rebaudioside A can be formed by glucosylation of the 13-OH of steviol which forms the 13-*O*-steviolmonoside, glucosylation of the C-2' of the 13-*O*-glucose of steviolmonoside which forms steviol-1,2-bioside, glucosylation of the C-19 carboxyl of steviol-1,2-bioside which forms stevioside, and glucosylation of the C-3' of the C-13-O-glucose of stevioside. The order in which each glucosylation reaction occurs can vary - see Figure 7. One UGT may be capable of catalyzing more than one conversion as set out in this scheme.

We have shown that conversion of steviol to rebaudioside M may be accomplished in a recombinant host by the expression of gene(s) encoding the following functional UGTs: UGT74G1, UGT85C2, UGT76G1 and UGT2.

Thus, a recombinant microorganism expressing these four UGTs can make rebaudioside M if it produces steviol or when fed steviol in the medium. Typically, one or more of these genes are recombinant genes that have been transformed into a microorganism that does not naturally possess them.

Examples of all of these enzmyes are set out in Table 1. A microorganism disclosed may comprise any combination of a UGT74G1, UGT85C2, UGT76G1 and UGT2. In Table 1 UGT64G1 sequences are indicated as UGT1 sequences, UGT74G1 sequences are indicated as UGT3 sequences and UGT76G1 sequences are indicated as UGT4 sequences. UGT2 sequences are indicated as UGT2 sequences in Table 1.

A recombinant microorganism disclosedcomprises a nucleotide sequence encoding a polypeptide capable of catalyzing the addition of a C-13-glucose to steviol. That is to say, a microorganism disclosedmay comprise a UGT which is capable of catalyzing a reaction in which steviol is converted to steviolmonoside.

Such a microorganism disclosedmay comprise a nucleotide sequence encoding a polypeptide having the activity shown by UDP-glycosyltransferase (UGT) UGT85C2, whereby the nucleotide sequence upon transformation of the microorganism confers on the cell the ability to convert steviol to steviolmonoside.

UGT85C2 activity is transfer of a glucose unit to the 13-OH of steviol. Thus, a suitable UGT85C2 may function as a uridine 5'-diphospho glucosyl: steviol 13-OH transferase, and a uridine 5'-diphospho glucosyl: steviol- 19-0- glucoside 13-OH transferase. A functional UGT85C2 polypeptides may also catalyze glucosyl transferase reactions that utilize steviol glycoside substrates other than steviol and steviol- 19-O-glucoside. Such sequences are indicated as UGT1 sequences in Table 1.

A recombinant microorganism disclosedalso comprises a nucleotide sequence encoding a polypeptide having UGT activity may comprise a nucleotide sequence encoding a polypeptide capable of catalyzing the addition of a C-13-glucose to steviol or steviolmonoside. That is to say, a microorganism disclosedmay comprise a UGT which is capable of catalyzing a reaction in which steviolmonoside is converted to steviolbioside. Accordingly, such a microorganism may be capable of converting steviolmonoside to steviolbioside. Expression of such a nucleotide sequence may confer on the microorganism the ability to produce at least steviolbioside.

A microorganism disclosedmay thus comprise a nucleotide sequence encoding a polypeptide having the activity shown by UDP-glycosyltransferase (UGT) UGT2, whereby the nucleotide sequence upon transformation of the microorganism confers on the cell the ability to convert steviolmonoside to steviolbioside.

A suitable UGT2 polypeptide functions as a uridine 5'-diphospho glucosyl: steviol- 13-O-glucoside transferase (also referred to as a steviol-13- monoglucoside 1,2-glucosylase), transferring a glucose moiety to the C-2' of the 13- O-glucose of the acceptor molecule, steviol- 13-O-glucoside. Typically, a suitable UGT2 polypeptide also functions as a uridine 5'-diphospho glucosyl: rubusoside transferase transferring a glucose moiety to the C-2' of the 13-O-glucose of the acceptor molecule, rubusoside.

Functional UGT2 polypeptides may also catalyze reactions that utilize steviol glycoside substrates other than steviol- 13-0-glucoside and rubusoside, e.g., functional UGT2 polypeptides may utilize stevioside as a substrate, transferring a glucose moiety to the C-2' of the 19-O-glucose residue to produce Rebaudioside E. A functional UGT2 polypeptides may also utilize Rebaudioside A as a substrate, transferring a glucose moiety to the C-2' of the 19-O-glucose residue to produce Rebaudioside D. However, a functional UGT2 polypeptide typically does not transfer a glucose moiety to steviol compounds having a 1,3-bound glucose at the C- 13 position, i.e., transfer of a glucose moiety to steviol 1,3-bioside and 1,3-stevioside does not occur. Functional UGT2 polypeptides may also transfer sugar moieties from donors other than uridine diphosphate glucose. For example, a functional UGT2 polypeptide may act as a uridine 5'-diphospho D-xylosyl: steviol- 13 -O-glucoside transferase, transferring a xylose moiety to the C-2' of the 13-O-glucose of the acceptor molecule, steviol- 13 -O-glucoside. As another example, a functional UGT2 polypeptide can act as a uridine 5'-diphospho L-rhamnosyl: steviol- 13-0- glucoside transferase, transferring a rhamnose moiety to the C-2' of the 13-O-glucose of the acceptor molecule, steviol-13-O-glucoside. Such sequences are indicated as UGT2 sequences in Table 1.

A recombinant microorganism disclosedalso comprises a nucleotide sequence encoding a polypeptide having UGT activity may comprise a nucleotide sequence encoding a polypeptide capable of catalyzing the addition of a C-19-glucose to steviolbioside. That is to say, a microorganism disclosedmay comprise a UGT which is capable of catalyzing a reaction in which steviolbioside is converted to stevioside. Accordingly, such a microorganism may be capable of converting steviolbioside to stevioside. Expression of such a nucleotide sequence may confer on the microorganism the ability to produce at least stevioside.

A microorganism disclosedmay thus also comprise a nucleotide sequence encoding a polypeptide having the activity shown by UDP-glycosyltransferase (UGT) UGT74G1, whereby the nucleotide sequence upon transformation of the microorganism confers on the cell the ability to convert steviolbioside to stevioside.

Suitable UGT74G1 polypeptides may be capable of transferring a glucose unit to the 13-OH or the 19-COOH, respectively, of steviol. A suitable UGT74G1 polypeptide may function as a uridine 5'-diphospho glucosyl: steviol 19-COOH transferase and a uridine 5'-diphospho glucosyl: steviol- 13-O-glucoside 19-COOH transferase. Functional UGT74G1 polypeptides also may catalyze glycosyl transferase reactions that utilize steviol glycoside substrates other than steviol and steviol- 13-O-glucoside, or that transfer sugar moieties from donors other than uridine diphosphate glucose. Such sequences are indicated as UGT1 sequences in Table 3.

A recombinant microorganism disclosedalso comprises a nucleotide sequence encoding a polypeptide capable of catalyzing glucosylation of the C-3' of the glucose at the C-13 position of stevioside. That is to say, a microorganism disclosedmay comprise a UGT which is capable of catalyzing a reaction in which stevioside to rebaudioside A. Accordingly, such a microorganism may be capable of converting stevioside to rebaudioside A. Expression of such a nucleotide sequence may confer on the microorganism the ability to produce at least rebaudioside A.

A microorganism disclosedmay thus also comprise a nucleotide sequence encoding a polypeptide having the activity shown by UDP-glycosyltransferase (UGT) UGT76G1, whereby the nucleotide sequence upon transformation of the microorganism confers on the cell the ability to convert stevioside to rebaudioside A.

A suitable UGT76G1 adds a glucose moiety to the C-3'of the C-13-O-glucose of the acceptor molecule, a steviol 1,2 glycoside. Thus, UGT76G1 functions, for example, as a uridine 5'-diphospho glucosyl: steviol 13-0-1,2 glucoside C-3 ' glucosyl transferase and a uridine 5'-diphospho glucosyl: steviol- 19-0-glucose, 13-0-1,2 bioside C-3' glucosyl transferase. Functional UGT76G1 polypeptides may also catalyze glucosyl transferase reactions that utilize steviol glycoside substrates that contain sugars other than glucose, e.g., steviol rhamnosides and steviol xylosides. Such sequences are indicated as UGT4 sequences in Table 1.

A microorganism disclosedcomprises nucleotide sequences encoding polypeptides having all four UGT activities described above. A given nucleic acid may encode a polypeptide having one or more of the above activities. For example, a nucleic acid encode for a polypeptide which has two, three or four of the activities set out above. Preferably, a recombinant microorganism disclosedcomprises UGT1, UGT2 and UGT3 and UGT4 activity.

A microorganism disclosedcomprises a nucleotide sequence encoding a polypeptide having UGT activity capable of catalyzing the glucosylation of stevioside or rebaudioside A. That is to say, a microorganism disclosedmay comprise a UGT which is capable of catalyzing a reaction in which stevioside or rebaudioside A is converted to rebaudioside D. Accordingly, such a microorganism may be capable of converting stevioside or rebaudioside A to rebaudioside D. Expression of such a nucleotide sequence may confer on the microorganism the ability to produce at least rebaudioside D.

We have shown that a microorganism expression a combination of UGT85C2, UGT2, UGT74G1 and UGT76G1 polypeptides is capable of rebaudioside M production.

A microorganism disclosedwhich comprises a nucleotide sequence encoding a polypeptide having UGT activity may comprise a nucleotide sequence encoding a polypeptide capable of catalyzing the glucosylation of stevioside. That is to say, a microorganism disclosedmay comprise a UGT which is capable of catalyzing a reaction in which stevioside is converted to rebaudioside E. Accordingly, such a microorganism may be capable of converting stevioside to rebaudioside E. Expression of such a nucleotide sequence may confer on the microorganism the ability to produce at least rebaudioside E.

A microorganism disclosedwhich comprises a nucleotide sequence encoding a polypeptide having UGT activity may comprise a nucleotide sequence encoding a polypeptide capable of catalyzing the glucosylation of rebaudioside E. That is to say, a microorganism disclosedmay comprise a UGT which is capable of catalyzing a reaction in which rebaudioside E is converted to rebaudioside D. Accordingly, such a microorganism may be capable of converting stevioside or rebaudioside A to rebaudioside D. Expression of such a nucleotide sequence may confer on the microorganism the ability to produce at least rebaudioside D.

A recombinant microorganism disclosedmay be capable of expressing a nucleotide sequence encoding a polypeptide having NADPH-cytochrome p450 reductase activity. That is to say, a recombinant microorganism disclosedmay comprise sequence encoding a polypeptide having NADPH-cytochrome p450 reductase activity.

For the purposes of the invention, a polypeptide having NADPH-Cytochrome P450 reductase activity (EC 1.6.2.4; also known as NADPH:ferrihemoprotein oxidoreductase, NADPH:hemoprotein oxidoreductase, NADPH:P450 oxidoreductase, P450 reductase, POR, CPR, CYPOR) is typically one which is a membrane-bound enzyme allowing electron transfer to cytochrome P450 in the microsome of the eukaryotic cell from a FAD- and FMN-containing enzyme NADPH:cytochrome P450 reductase (POR; EC 1.6.2.4).

Preferably, a recombinant microorganism disclosed is capable of expressing one or more of:
a. a nucleotide sequence encoding a polypeptide having NADPH-cytochrome p450 reductase activity, wherein said nucleotide sequence comprises:
   i. a nucleotide sequence encoding a polypeptide having NADPH-cytochrome p450 reductase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 54, 56, 58 or 78;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 53, 55, 57 or 77;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,

Preferably, a recombinant microorganism disclosedis one which is capable of expressing one or more of:
a. a nucleotide sequence encoding a polypeptide having ent-copalyl pyrophosphate synthase activity, wherein said nucleotide sequence comprises:
   i. a nucleotide sequence encoding a polypeptide having ent-copalyl pyrophosphate synthase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 2, 4, 6, 8, 18, 20, 60 or 62;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 1, 3, 5, 7, 17, 19, 59 or 61, 141, 142, 151, 152, 153, 154, 159, 160, 182 or 184;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
b. a nucleotide sequence encoding a polypeptide having ent-Kaurene synthase activity, wherein said nucleotide sequence comprises:
   i. a nucleotide sequence encoding a polypeptide having ent-Kaurene synthase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 10, 12, 14, 16, 18, 20, 64 or 66;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 9, 11, 13, 15, 17, 19, 63, 65, 143, 144, 155, 156, 157, 158, 159, 160, 183 or 184;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
c. a nucleotide sequence encoding a polypeptide having ent-Kaurene oxidase activity, wherein said nucleotide sequence comprises:
   i. a nucleotide sequence encoding a polypeptide having ent-Kaurene oxidase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 22, 24, 26, 68 or 86;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 21, 23, 25, 67, 85, 145, 161, 162, 163, 180 or 186;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code; or
d. a nucleotide sequence encoding a polypeptide having kaurenoic acid 13-hydroxylase activity, wherein said nucleotide sequence comprises:
   i. a nucleotide sequence encoding a polypeptide having kaurenoic acid 13-hydroxylase activity, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 28, 30, 32, 34, 70, 90, 92, 94, 96 or 98;
   ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 27, 29, 31, 33, 69, 89, 91, 93, 95, 97, 146, 164, 165, 166, 167 or 185;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code.

In a recombinant microorganism disclosed, which is capable of expressing a nucleotide sequence encoding a polypeptide capable of catalyzing the addition of a C-13-glucose to steviol (addition of glucose to the C-13 position of steviol), said nucleotide may comprise:
i. a nucleotide sequence encoding a polypeptide capable of catalyzing the addition of a C-13-glucose to steviol, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 36, 38 or 72;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 35, 37, 71, 147, 168, 169 or 189;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism disclosed, which is capable of expressing a nucleotide sequence encoding a polypeptide capable of catalyzing the addition of a glucose at the C-13 position of steviolmonoside (this typically indicates glucosylation of the C-2' of the C-13-glucose/13-O-glucose of steviolmonoside), said nucleotide sequence may comprise:
i. a nucleotide sequence encoding a polypeptide capable of catalyzing the addition of a C-13-glucose to steviol or steviolmonoside, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 88, 100, 102, 104, 106, 108, 110 or 112;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 87, 99, 101, 103, 105, 107, 109, 111, 181 or 192;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism disclosed, which is capable of expressing a nucleotide sequence encoding a polypeptide capable of catalyzing the addition of a glucose at the C-19 position of steviolbioside, said nucleotide sequence may comprise:
i. a nucleotide sequence encoding a polypeptide capable of catalyzing the addition of a glucose at the C-19 position of steviolbioside, said polypeptide comprising an amino acid sequence that has at least about 20% sequence identity with the amino acid sequence of SEQ ID NOs: 40, 42, 44, 46, 48 or 74;
ii. a nucleotide sequence that has at least about 15% sequence identity with the nucleotide sequence of SEQ ID NOs: 39, 41, 43, 45, 47, 73, 148, 170, 171, 172, 173, 174 or 190 ;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism disclosed, which expresses a nucleotide sequence encoding a polypeptide capable of catalyzing glucosylation of the C-3' of the glucose at the C-13 position of stevioside, said nucleotide sequence may comprise:
i. a nucleotide sequence encoding a polypeptide capable of catalyzing glucosylation of the C-3' of the glucose at the C-13 position of stevioside, said polypeptide comprising an amino acid sequence that has at least about 20%, preferably at least 25, 30, 40, 50, 55, 60, 65, 70, 75, 80, 85, 90, 95, 96, 97, 98, or 99%, sequence identity with the amino acid sequence of SEQ ID NOs: 50, 52 or 76;
ii. a nucleotide sequence that has at least about 15%, preferably at least 20, 25, 30, 40, 50, 55, 60, 65, 70, 75, 80, 85, 90, 95, 96, 97, 98, or 99%, sequence identity with the nucleotide sequence of SEQ ID NOs: 49, 51, 75, 149, 175, 176 or 191;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

In a recombinant microorganism disclosed, which expresses a nucleotide sequence encoding a polypeptide capable of catalysing one or more of: the glucosylation of stevioside or rebaudioside A to rebaudioside D; the glucosylation of stevioside to rebaudioside E; the glucosylation of rebaudioside E to rebaudioside D; or the glucosylation of rebaudioside D to rebaudioside M, said nucleotide sequence may comprise:
i. a nucleotide sequence encoding a polypeptide capable of catalysing one or more of: the glucosylation of stevioside or rebaudioside A to rebaudioside D; the glucosylation of stevioside to rebaudioside E; the glucosylation of rebaudioside E to rebaudioside D; or the glucosylation of rebaudioside D to rebaudioside M, said polypeptide comprising an amino acid sequence that has at least about 20% sequence identity with the amino acid sequence of SEQ ID NOs: 88, 100, 102, 104, 106, 108, 110, 112;
ii. a nucleotide sequence that has at least about 15% sequence identity with the nucleotide sequence of SEQ ID NOs: 87, 99, 101, 103, 105, 107, 109, 111, 181 or 192;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

A microorganism disclosed, may be one in which the ability of the microorganism to produce geranylgeranyl pyrophosphate (GGPP) is upregulated. Upregulated in the context of this invention implies that the microorganism produces more GGPP than an equivalent non-transformed strain.

Accordingly, a microorganism disclosedmay comprise one or more nucleotide sequence(s) encoding hydroxymethylglutaryl-CoA reductase, farnesyl-pyrophosphate synthetase and geranylgeranyl diphosphate synthase, whereby the nucleotide sequence(s) upon transformation of the microorganism confer(s) on the microorganism the ability to produce elevated levels of GGPP.

Preferably, a microorganism disclosedis one which is capable of expressing one or more of:
a. a nucleotide sequence encoding a polypeptide having hydroxymethylglutaryl-CoA reductase activity, wherein said nucleotide sequence comprises:
   i. a nucleotide sequence encoding a polypeptide having hydroxymethylglutaryl-CoA reductase activity, said polypeptide comprising an amino acid sequence that has at least about 20% sequence identity with the amino acid sequence of SEQ ID NO: 80;
   ii. a nucleotide sequence that has at least about 15% sequence identity with the nucleotide sequence of SEQ ID NO: 79;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code,
b. a nucleotide sequence encoding a polypeptide having farnesyl-pyrophosphate synthetase activity, wherein said nucleotide sequence comprises:
   i. a nucleotide sequence encoding a polypeptide having farnesyl-pyrophosphate synthetase activity, said polypeptide comprising an amino acid sequence that has at least about 20% sequence identity with the amino acid sequence of SEQ ID NO: 82;
   ii. a nucleotide sequence that has at least about 15% sequence identity with the nucleotide sequence of SEQ ID NOs: 81;
   iii. a nucleotide sequence the complementary strand of which hybridizes to a nucleic acid molecule of sequence of (i) or (ii); or
   iv. a nucleotide sequence which differs from the sequence of a nucleic acid molecule of (iii) due to the degeneracy of the genetic code; or
c. a nucleotide sequence encoding a polypeptide having geranylgeranyl diphosphate synthase activity, wherein said nucleotide sequence comprises:
   i. a nucleotide sequence encoding a polypeptide having geranylgeranyl diphosphate synthase activity, said polypeptide comprising an amino acid sequence that has at least about 20% sequence identity with the amino acid sequence of SEQ ID NO: 84;
   ii. a nucleotide sequence that has at least about 15% sequence identity with the nucleotide sequence of SEQ ID NOs: 83;
   iii. a nucleotide sequence the complementary strand of which hybridizes to a nucleic acid molecule of sequence of (i) or (ii); or
   iv. a nucleotide sequence which differs from the sequence of a nucleic acid molecule of (i), (ii) or (iii) due to the degeneracy of the genetic code.

The invention relates to a recombinant microorganism. A microorganism or microbe, for the purposes of this invention, is typically an organism that is not visible to the human eye (i.e. microscopic). A microorganism may be from bacteria, fungi, archaea or protists. Typically a microorganism will be a single-celled or unicellular organism.

As used herein a recombinant microorganism is defined as a microorganism which is genetically modified or transformed/transfected with one or more of the nucleotide sequences as defined herein. The presence of the one or more such nucleotide sequences alters the ability of the microorganism to produce a diterpene or diterpene glycoside, in particular steviol or steviol glycoside. A microorganism that is not transformed/transfected or genetically modified, is not a recombinant microorganism and does typically not comprise one or more of the nucleotide sequences enabling the cell to produce a diterpene or diterpene glycoside. Hence, a non-transformed/non-transfected microorganism is typically a microorganism that does not naturally produce a diterpene, although a microorganism which naturally produces a diterpene or diterpene glycoside and which has been modified according to the invention (and which thus has an altered ability to produce a diterpene/diterpene gylcoside) is considered a recombinant microorganism according to the invention.

Sequence identity is herein defined as a relationship between two or more amino acid (polypeptide or protein) sequences or two or more nucleic acid (polynucleotide) sequences, as determined by comparing the sequences. Usually, sequence identities or similarities are compared over the whole length of the sequences compared. In the art, "identity" also means the degree of sequence relatedness between amino acid or nucleic acid sequences, as the case may be, as determined by the match between strings of such sequences. "Identity" and "similarity" can be readily calculated by various methods, known to those skilled in the art. Preferred methods to determine identity are designed to give the largest match between the sequences tested. Typically then, identities and similarities are calculated over the entire length of the sequences being compared. Methods to determine identity and similarity are codified in publicly available computer programs. Preferred computer program methods to determine identity and similarity between two sequences include e.g. the BestFit, BLASTP, BLASTN, and FASTA (Altschul, S. F. et al., J. Mol. Biol. 215:403-410 (1990), publicly available from NCBI and other sources (BLAST Manual, Altschul, S., et al., NCBI NLM NIH Bethesda, MD 20894). Preferred parameters for amino acid sequences comparison using BLASTP are gap open 10.0, gap extend 0.5, Blosum 62 matrix. Preferred parameters for nucleic acid sequences comparison using BLASTP are gap open 10.0, gap extend 0.5, DNA full matrix (DNA identity matrix).

Nucleotide sequences encoding the enzymes expressed in the cell disclosed may also be defined by their capability to hybridize with the nucleotide sequences of SEQ ID NO.'s 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81 or 84 ir any other sequence mentioned herein respectively, under moderate, or preferably under stringent hybridisation conditions. Stringent hybridisation conditions are herein defined as conditions that allow a nucleic acid sequence of at least about 25, preferably about 50 nucleotides, 75 or 100 and most preferably of about 200 or more nucleotides, to hybridise at a temperature of about 65°C in a solution comprising about 1 M salt, preferably 6 x SSC or any other solution having a comparable ionic strength, and washing at 65°C in a solution comprising about 0.1 M salt, or less, preferably 0.2 x SSC or any other solution having a comparable ionic strength. Preferably, the hybridisation is performed overnight, i.e. at least for 10 hours and preferably washing is performed for at least one hour with at least two changes of the washing solution. These conditions will usually allow the specific hybridisation of sequences having about 90% or more sequence identity.

Moderate conditions are herein defined as conditions that allow a nucleic acid sequences of at least 50 nucleotides, preferably of about 200 or more nucleotides, to hybridise at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridisation is performed overnight, i.e. at least for 10 hours, and preferably washing is performed for at least one hour with at least two changes of the washing solution. These conditions will usually allow the specific hybridisation of sequences having up to 50% sequence identity. The person skilled in the art will be able to modify these hybridisation conditions in order to specifically identify sequences varying in identity between 50% and 90%.

The nucleotide sequences encoding an ent-copalyl pyrophosphate synthase; ent-Kaurene synthase; ent-Kaurene oxidase; kaurenoic acid 13-hydroxylase; UGT; hydroxymethylglutaryl-CoA reductase, farnesyl-pyrophosphate synthetase; geranylgeranyl diphosphate synthase; NADPH-cytochrome p450 reductase, may be from prokaryotic or eukaryotic origin.

A nucleotide sequence encoding an ent-copalyl pyrophosphate synthase may for instance comprise a sequence as set out in SEQ ID. NO: 1, 3, 5, 7, 17, 19, 59, 61, 141, 142, 151, 152, 153, 154, 159, 160, 182 or 184.

A nucleotide sequence encoding an ent-Kaurene synthase may for instance comprise a sequence as set out in SEQ ID. NO: 9, 11, 13, 15, 17, 19, 63, 65, 143, 144, 155, 156, 157, 158, 159, 160, 183 or 184.

A nucleotide sequence encoding an ent-Kaurene oxidase may for instance comprise a sequence as set out in SEQ ID. NO: 21, 23, 25, 67, 85, 145, 161, 162, 163, 180 or 186. A preferred KO is the polypeptide encoded by the nucleic acid set out in SEQ ID NO: 85.

A nucleotide sequence encoding a kaurenoic acid 13-hydroxylase may for instance comprise a sequence as set out in SEQ ID. NO: 27, 29, 31, 33, 69, 89, 91, 93, 95, 97, 146, 164, 165, 166, 167 or 185. A preferred KAH sequence is the polypeptide encoded by the nucleic acid set out in SEQ ID NO: 33.

A further preferred recombinant microorganism disclosedmay express a combination of the polypeptides encoded by SEQ ID NO: 85 and SEQ ID NO: 33 or a variant of either thereof as herein described. A preferred recombinant microorganism disclosedmay expression the combination of sequences set out in Table 8 (in combination with any UGT2, but in particular that encoded by SEQ ID NO: 87).

A nucleotide sequence encoding a UGT may for instance comprise a sequence as set out in SEQ ID. NO: 35, 37, 39, 41, 43, 45, 47, 49, 51, 71, 73, 75, 168, 169, 170, 171, 172, 173, 174, 175, 176, 147, 148, 149, 87, 181, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 189, 190, 191 or 192.

A nucleotide sequence encoding a hydroxymethylglutaryl-CoA reductase may for instance comprise a sequence as set out in SEQ ID. NO: 79.

A nucleotide sequence encoding a farnesyl-pyrophosphate synthetase may for instance comprise a sequence as set out in SEQ ID. NO: 81.

A nucleotide sequence encoding a geranylgeranyl diphosphate synthase may for instance comprise a sequence as set out in SEQ ID. NO:83.

A nucleotide sequence encoding a NADPH-cytochrome p450 reductase may for instance comprise a sequence as set out in SEQ ID. NO: 53, 55, 57 or 77.

In the case of the UGT sequences, combinations of at least one from each of: (i) SEQ ID NOs: 35, 37, 168, 169, 71, 147 or 189; (ii) SEQ ID NOs: 87, 99, 101, 103, 105, 107, 109, 111, 181 or 192; (iii) SEQ ID NOs: 39, 41, 43, 45, 47, 170, 171, 172, 173, 174, 73, 148 or 190; and (iv) SEQ ID NOs: 49, 51, 175, 176, 75, 149 or 191 may be preferred. Typically, at least one UGT from group (i) may be used. If at least one UGT from group (iii) is used, generally at least one UGT from group (i) is also used. If at least one UGT from group (iv) is used, generally at least one UGT from group (i) and at least one UGT from group (iii) is used. Typically, at least one UGT form group (ii) is used.

A sequence which has at least about 10%, about 15%, about 20%, preferably at least about 25%, about 30%, about 40%, about 50%, about 55%, about 60%, about 65%, about 70%, about 75%, about 80%, about 85%, about 90%, about 95%, about 96%, about 97%, about 98%, or about 99% sequence identity with a sequence as mentioned may be used in the invention.

To increase the likelihood that the introduced enzymes are expressed in active form in a eukaryotic cell disclosed, the corresponding encoding nucleotide sequence may be adapted to optimise its codon usage to that of the chosen eukaryote host cell. The adaptiveness of the nucleotide sequences encoding the enzymes to the codon usage of the chosen host cell may be expressed as codon adaptation index (CAI). The codon adaptation index is herein defined as a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li, 1987, Nucleic Acids Research 15: 1281-1295; also see: Jansen et al., 2003, Nucleic Acids Res. 31(8):2242-51). An adapted nucleotide sequence preferably has a CAI of at least 0.2, 0.3, 0.4, 0.5, 0.6 or 0.7.

In an embodiment the eukaryotic cell disclosedis genetically modified with (a) nucleotide sequence(s) which is (are) adapted to the codon usage of the eukaryotic cell using codon pair optimisation technology as disclosed in PCT/EP2007/05594. Codon-pair optimisation is a method for producing a polypeptide in a host cell, wherein the nucleotide sequences encoding the polypeptide have been modified with respect to their codon-usage, in particular the codon-pairs that are used, to obtain improved expression of the nucleotide sequence encoding the polypeptide and/or improved production of the polypeptide. Codon pairs are defined as a set of two subsequent triplets (codons) in a coding sequence.

Further improvement of the activity of the enzymes *in vivo* in a eukaryotic host cell disclosed, can be obtained by well-known methods like error prone PCR or directed evolution. A preferred method of directed evolution is described in WO03010183 and WO03010311.

The microorganism disclosedmay be any suitable host cell from microbial origin. Preferably, the host cell is a yeast or a filamentous fungus. More preferably, the host cell belongs to one of the genera *Saccharomyces, Aspergillus, Penicillium, Pichia, Kluyveromyces, Yarrowia, Candida, Hansenula, Humicola, Torulaspora, Trichosporon, Brettanomyces, Pachysolen* or *Yamadazyma* or *Zygosaccharomyces.*

A more preferred microorganism disclosed belongs to the species *Aspergillus niger, Penicillium chrysogenum, Pichia stipidis, Kluyveromyces marxianus, K. lactis, K. thermotolerans, Yarrowia lipolytica, Candida sonorensis, C. glabrata, Hansenula polymorpha, Torulaspora delbrueckii, Brettanomyces bruxellensis, Zygosaccharomyces bailii, Saccharomyces uvarum, Saccharomyces bayanus* or *Saccharomyces cerevisiae* species. Preferably, the eukaryotic cell is a *Saccharomyces cerevisiae.*

A recombinant yeast cell disclosedmay be modified so that the ERG9 gene is down-regulated and or the ERG5/ERG6 genes are deleted. Corresponding genes may be modified in this way in other microorganisms.

Such a microorganism may be transformed as set out herein, whereby the nucleotide sequence(s) with which the microorganism is transformed confer(s) on the cell the ability to produce RebM.

A preferred microorganism disclosedis a yeast such as a *Saccharomyces cerevisiae* or *Yarrowia lipolytica* cell. A recombinant microorganism disclosed, such as a recombinant *Saccharomyces cerevisiae* cell or *Yarrowia lipolytica* cell may comprise one or more nucleotide sequence(s) from each of the following groups;
(i) SEQ ID. NO: 1, 3, 5, 7, 17, 19, 59, 61, 141, 142, 152, 153, 154, 159, 160, 182 or 184.
(ii) SEQ ID. NO: 9, 11, 13, 15, 17, 19, 63, 65, 143, 144, 155, 156, 157, 158, 159, 160, 183 or 184.
(iii) SEQ ID. NO: 21, 23, 25, 67 85, 145, 161, 162, 163, 180 or 186.
(iv) SEQ ID. NO: 27, 29, 31, 33, 69, 89, 91, 93, 95, 97, 146, 164, 165, 166, 167 or 185.

Such a microorganism will typically also comprise one or more nucleotide sequence(s) as set out in SEQ ID. NO: 53, 55, 57 or 77.

Such a microorganism may also comprise one or more nucleotide sequences as set out in 35, 37, 39, 41, 43, 45, 47, 49, 51, 71, 73, 75, 168, 169, 170, 171, 172, 173, 174, 175, 176, 147, 148, 149, 87, 181, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 189, 190, 191 or 192. In the case of these sequences, combinations of at least one from each of (i) SEQ ID NOs: 35, 37, 168, 169, 71, 147 or 189; (ii) SEQ ID NOs: 87, 99, 101, 103, 105, 107, 109, 111, 181 or 192; (iii) SEQ ID NOs: 39, 41, 43, 45, 47, 170, 171, 172, 173, 174, 73, 148 or 190; and (iv) SEQ ID NOs: 49, 51, 175, 176, 75, 149 or 191 may be preferred. Typically, at least one UGT from group (i) may be used. If at least one UGT from group (iii) is used, generally at least one UGT from group (i) is also used. If at least one UGT from group (iv) is used, generally at least one UGT from group (i) and at least one UGT from group (iii) is used. Typically, at least one UGT form group (ii) is used.

Such a microorganism may also comprise the following nucleotide sequences: SEQ ID. NO: 79; SEQ ID. NO: 81; and SEQ ID. NO: 83.

For each sequence set out above (or any sequence mentioned herein), a variant having at least about 15%, preferably at least about 20, about 25, about 30, about 40, about 50, about 55, about 60, about 65, about 70, about 75, about 80, about 85, about 90, about 95, about 96, about 97, about 98, or about 99%, sequence identity with the stated sequence may be used.

The nucleotide sequences encoding the ent-copalyl pyrophosphate synthase, ent-Kaurene synthase, ent-Kaurene oxidase, kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase may be ligated into one or more nucleic acid constructs to facilitate the transformation of the microorganism disclosed.

A nucleic acid construct may be a plasmid carrying the genes encoding enzymes of the RebM pathway as described above, or a nucleic acid construct may comprise two or three plasmids carrying each three or two genes, respectively, encoding the enzymes of the diterpene pathway distributed in any appropriate way.

Any suitable plasmid may be used, for instance a low copy plasmid or a high copy plasmid.

It may be possible that the enzymes selected from the group consisting of ent-copalyl pyrophosphate synthase, ent-Kaurene synthase, ent-Kaurene oxidase, and kaurenoic acid 13-hydroxylase, UGTs, hydroxymethylglutaryl-CoA reductase, farnesyl-pyrophosphate synthetase, geranylgeranyl diphosphate synthase and NADPH-cytochrome p450 reductase are native to the host microorganism and that transformation with one or more of the nucleotide sequences encoding these enzymes may not be required to confer the host cell the ability to produce a diterpene or diterpene glycosidase. Further improvement of diterpene/diterpene glycosidase production by the host microorganism may be obtained by classical strain improvement.

The nucleic acid construct may be maintained episomally and thus comprise a sequence for autonomous replication, such as an autosomal replication sequence sequence. If the host cell is of fungal origin, a suitable episomal nucleic acid construct may e.g. be based on the yeast 2µ or pKD1 plasmids (Gleer et al., 1991, Biotechnology 9: 968-975), or the AMA plasmids (Fierro et al., 1995, Curr Genet. 29:482-489).

Alternatively, each nucleic acid construct may be integrated in one or more copies into the genome of the host cell. Integration into the host cell's genome may occur at random by non-homologous recombination but preferably the nucleic acid construct may be integrated into the host cell's genome by homologous recombination as is well known in the art (see e.g. WO90/14423, EP-A-0481008, EP-A-0635 574 and US 6,265,186).

Optionally, a selectable marker may be present in the nucleic acid construct. As used herein, the term "marker" refers to a gene encoding a trait or a phenotype which permits the selection of, or the screening for, a microorganism containing the marker. The marker gene may be an antibiotic resistance gene whereby the appropriate antibiotic can be used to select for transformed cells from among cells that are not transformed. Alternatively or also, non-antibiotic resistance markers are used, such as auxotrophic markers (URA3, TRP1, LEU2). The host cells transformed with the nucleic acid constructs may be marker gene free. Methods for constructing recombinant marker gene free microbial host cells are disclosed in EP-A-0 635 574 and are based on the use of bidirectional markers. Alternatively, a screenable marker such as Green Fluorescent Protein, *lac*Z, luciferase, chloramphenicol acetyltransferase, beta-glucuronidase may be incorporated into the nucleic acid constructs disclosedallowing to screen for transformed cells. A preferred marker-free method for the introduction of heterologous polynucleotides is described in WO0540186.

In an embodiment, the nucleotide sequences encoding ent-copalyl pyrophosphate synthase, ent-Kaurene synthase, ent-Kaurene oxidase, and kaurenoic acid 13-hydroxylase, UGTs, hydroxymethylglutaryl-CoA reductase, farnesyl-pyrophosphate synthetase ,geranylgeranyl diphosphate synthase and NADPH-cytochrome p450 reductase, are each operably linked to a promoter that causes sufficient expression of the corresponding nucleotide sequences in the eukaryotic cell disclosedto confer to the cell the ability to produce RebM.

As used herein, the term "operably linked" refers to a linkage of polynucleotide elements (or coding sequences or nucleic acid sequence) in a functional relationship. A nucleic acid sequence is "operably linked" when it is placed into a functional relationship with another nucleic acid sequence. For instance, a promoter or enhancer is operably linked to a coding sequence if it affects the transcription of the coding sequence.

As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skilled in the art to act directly or indirectly to regulate the amount of transcription from the promoter. A "constitutive" promoter is a promoter that is active under most environmental and developmental conditions. An "inducible" promoter is a promoter that is active under environmental or developmental regulation.

The promoter that could be used to achieve the expression of the nucleotide sequences coding for an enzyme as defined herein above, may be not native to the nucleotide sequence coding for the enzyme to be expressed, i.e. a promoter that is heterologous to the nucleotide sequence (coding sequence) to which it is operably linked. Preferably, the promoter is homologous, i.e. endogenous to the host cell

Suitable promoters in microorganisms disclosedmay be GAL7, GAL10, or GAL 1, CYC1, HIS3, ADH1, PGL, PH05, GAPDH, ADC1, TRP1, URA3, LEU2, ENO, TPI, and AOX1. Other suitable promoters include PDC, GPD1, PGK1, TEF1, and TDH. Further suitable promoters are set out in the Examples.

Any terminator, which is functional in the cell, may be used in the disclosure. Preferred terminators are obtained from natural genes of the host cell. Suitable terminator sequences are well known in the art. Preferably, such terminators are combined with mutations that prevent nonsense mediated mRNA decay in the host cell disclosed (see for example: Shirley et al., 2002, Genetics 161:1465-1482).

Nucleotide sequences used in the invention may include sequences which target them to desired compartments of the microorganism. For example, in a preferred microorganism disclosed, all nucleotide sequences, except for ent-Kaurene oxidase, kaurenoic acid 13-hydroxylase and NADPH-cytochrome p450 reductase encoding sequences may be targeted to the cytosol. This approach may be used in a yeast cell.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain.

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which it is introduced, but have been obtained from another cell or synthetically or recombinantly produced.

Typically, recombinant microorganism disclosedwill comprise heterologous nucleotide sequences. Alternatively, a recombinant microorganism disclosed may comprise entirely homologous sequence which has been modified as set out herein so that the microorganism produces increased amounts of RebM in comparison to a non-modified version of the same microorganism.

One or more enzymes of the diterpene pathway as described herein may be overexpressed to achieve a sufficient diterpene production by the cell.

There are various means available in the art for overexpression of enzymes in the host cells disclosed. In particular, an enzyme may be overexpressed by increasing the copy number of the gene coding for the enzyme in the host cell, e.g. by integrating additional copies of the gene in the host cell's genome.

A preferred host cell disclosedmay be a recombinant cell which is naturally capable of producing GGPP.

A recombinant microorganism disclosedmay be able to grow on any suitable carbon source known in the art and convert it to RebM. The recombinant microorganism may be able to convert directly plant biomass, celluloses, hemicelluloses, pectines, rhamnose, galactose, fucose, maltose, maltodextrines, ribose, ribulose, or starch, starch derivatives, sucrose, lactose and glycerol. Hence, a preferred host organism expresses enzymes such as cellulases (endocellulases and exocellulases) and hemicellulases (e.g. endo- and exo-xylanases, arabinases) necessary for the conversion of cellulose into glucose monomers and hemicellulose into xylose and arabinose monomers, pectinases able to convert pectines into glucuronic acid and galacturonic acid or amylases to convert starch into glucose monomers. Preferably, the host cell is able to convert a carbon source selected from the group consisting of glucose, xylose, arabinose, sucrose, lactose and glycerol. The host cell may for instance be a eukaryotic host cell as described in WO03/062430, WO06/009434, EP1499708B1, WO2006096130 or WO04/099381.

Further disclosed is a process for the production of RebM comprising fermenting a transformed eukaryotic cell disclosed in a suitable fermentation medium, and optionally recovering the RebM.

The fermentation medium used in the process for the production of RebM may be any suitable fermentation medium which allows growth of a particular eukaryotic host cell. The essential elements of the fermentation medium are known to the person skilled in the art and may be adapted to the host cell selected.

Preferably, the fermentation medium comprises a carbon source selected from the group consisting of plant biomass, celluloses, hemicelluloses, pectines, rhamnose, galactose, fucose, fructose, maltose, maltodextrines, ribose, ribulose, or starch, starch derivatives, sucrose, lactose, fatty acids, triglycerides and glycerol. Preferably, the fermentation medium also comprises a nitrogen source such as ureum, or an ammonium salt such as ammonium sulphate, ammonium chloride, ammoniumnitrate or ammonium phosphate.

The fermentation process disclosedmay be carried out in batch, fed-batch or continuous mode. A separate hydrolysis and fermentation (SHF) process or a simultaneous saccharification and fermentation (SSF) process may also be applied. A combination of these fermentation process modes may also be possible for optimal productivity. A SSF process may be particularly attractive if starch, cellulose, hemicelluose or pectin is used as a carbon source in the fermentation process, where it may be necessary to add hydrolytic enzymes, such as cellulases, hemicellulases or pectinases to hydrolyse the substrate.

The recombinant microorganism used in the process for the preparation of RebM may be any suitable microorganism as defined herein above. It may be advantageous to use a recombinant eukaryotic microorganism disclosedin the process for the production of RebM, because most eukaryotic cells do not require sterile conditions for propagation and are insensitive to bacteriophage infections. In addition, eukaryotic host cells may be grown at low pH to prevent bacterial contamination.

The recombinant microorganism disclosedmay be a facultative anaerobic microorganism. A facultative anaerobic microorganism can be propagated aerobically to a high cell concentration. This anaerobic phase can then be carried out at high cell density which reduces the fermentation volume required substantially, and may minimize the risk of contamination with aerobic microorganisms.

The fermentation process for the production of a diterpene disclosed may be an aerobic or an anaerobic fermentation process.

An anaerobic fermentation process may be herein defined as a fermentation process run in the absence of oxygen or in which substantially no oxygen is consumed, preferably less than 5, 2.5 or 1 mmol/L/h, and wherein organic molecules serve as both electron donor and electron acceptors. The fermentation process disclosed may also first be run under aerobic conditions and subsequently under anaerobic conditions.

The fermentation process may also be run under oxygen-limited, or micro-aerobical, conditions. Alternatively, the fermentation process may first be run under aerobic conditions and subsequently under oxygen-limited conditions. An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used.

The production of a diterpene in the process disclosed may occur during the growth phase of the host cell, during the stationary (steady state) phase or during both phases. It may be possible to run the fermentation process at different temperatures.

The process for the production of RebM may be run at a temperature which is optimal for the eukaryotic cell. The optimum growth temperature may differ for each transformed eukaryotic cell and is known to the person skilled in the art. The optimum temperature might be higher than optimal for wild type organisms to grow the organism efficiently under non-sterile conditions under minimal infection sensitivity and lowest cooling cost. Alternatively, the process may be carried out at a temperature which is not optimal for growth of the recombinant microorganism.

The process for the production of RebM disclosedmay be carried out at any suitable pH value. If the recombinant microorganism is yeast, the pH in the fermentation medium preferably has a value of below 6, preferably below 5,5, preferably below 5, preferably below 4,5, preferably below 4, preferably below pH 3,5 or below pH 3,0, or below pH 2,5, preferably above pH 2. An advantage of carrying out the fermentation at these low pH values is that growth of contaminant bacteria in the fermentation medium may be prevented.

Such a process may be carried out on an industrial scale.

The product of such a process is rebaudioside M.

Recovery of RebM from the fermentation medium may be performed by known methods in the art, for instance by distillation, vacuum extraction, solvent extraction, or evaporation.

In the process for the production of RebM disclosed, it may be possible to achieve a concentration of above 5 mg/l fermentation broth, preferably above 10 mg/l, preferably above 20 mg/l, preferably above 30 mg/l fermentation broth, preferably above 40 mg/l, more preferably above 50 mg/l, preferably above 60 mg/l, preferably above 70, preferably above 80 mg/l, preferably above 100 mg/l, preferably above 1 g/l, preferably above 5 g/l, preferably above 10 g/l, but usually below 70 g/l.

Also disclosed is a fermentation broth comprising RebM obtainable by the process disclosed.

In the event that RebM is expressed within the microorganism, such cells may need to be treated so as to release RebM. Preferentially, RebM is produced extracellularly

ThAlso disclosed is a method for converting a first glycosylated diterpene into a second glycosylated diterpene, which method comprises:
contacting said first glycosylated diterpene with a microorganism as herein described, a cell free extract derived from such a microorganism or an enzyme preparation derived from either thereof,
thereby to convert the first glycosylated diterpene into the second glycosylated diterpene.

The second glycosylated diterpene may be rebaudioside A, rebuadioside D or rebaudioside M. In particular, the method may be carried out in a format such that the first glycosylated diterpene is steviol, rebaudioside A or rebaudioside D and, preferably, the second glycosylated diterpene is rebaudioside M.

That is to say, disclosed is a method of bioconversion or biotransformation.

RebM produced by the fermentation process disclosed may be used in any application known for such compounds. In particular, they may for instance be used as a sweetener, for example in a food or a beverage. For example RebM may be formulated in soft drinks, as a tabletop sweetener, chewing gum, dairy product such as yoghurt (eg. plain yoghurt), cake, cereal or cereal-based food, nutraceutical, pharmaceutical, edible gel, confectionery product, cosmetic, toothpastes or other oral cavity composition, etc. In addition, RebM can be used as a sweetener not only for drinks, foodstuffs, and other products dedicated for human consumption, but also in animal feed and fodder with improved characteristics.

Accordingly, disclosed is, *inter alia,* a foodstuff, feed or beverage which comprises a diterpene or glycosylated prepared according to a process disclosed.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

The RebM obtained can be used in dry or liquid forms. It can be added before or after heat treatment of food products. The amount of the sweetener depends on the purpose of usage. It can be added alone or in the combination with other compounds.

Compounds produced according to the method disclosedmay be blended with one or more further non-calorific or calorific sweeteners. Such blending may be used to improve flavour or temporal profile or stability. A wide range of both non-calorific and calorific sweeteners may be suitable for blending with RebM. For example, non-calorific sweeteners such as mogroside, monatin, aspartame, acesulfame salts, cyclamate, sucralose, saccharin salts or erythritol. Calorific sweeteners suitable for blending with RebM include sugar alcohols and carbohydrates such as sucrose, glucose, fructose and HFCS. Sweet tasting amino acids such as glycine, alanine or serine may also be used.

The RebM can be used in the combination with a sweetener suppressor, such as a natural sweetener suppressor. It may be combined with an umami taste enhancer, such as an amino acid or a salt thereof.

RebM can be combined with a polyol or sugar alcohol, a carbohydrate, a physiologically active substance or functional ingredient (for example a carotenoid, dietary fiber, fatty acid, saponin, antioxidant, nutraceutical, flavonoid, isothiocyanate, phenol, plant sterol or stanol (phytosterols and phytostanols), a polyols, a prebiotic, a probiotic, a phytoestrogen, soy protein, sulfides/thiols, amino acids, a protein, a vitamin, a mineral, and/or a substance classified based on a health benefits, such as cardiovascular, cholesterol-reducing or anti-inflammatory.

A composition with RebM may include a flavoring agent, an aroma component, a nucleotide, an organic acid, an organic acid salt, an inorganic acid, a bitter compound, a protein or protein hydrolyzate, a surfactant, a flavonoid, an astringent compound, a vitamin, a dietary fiber, an antioxidant, a fatty acid and/or a salt.

RebM disclosedmay be applied as a high intensity sweetener to produce zero calorie, reduced calorie or diabetic beverages and food products with improved taste characteristics. Also it can be used in drinks, foodstuffs, pharmaceuticals, and other products in which sugar cannot be used.

In addition, RebM disclosedmay be used as a sweetener not only for drinks, foodstuffs, and other products dedicated for human consumption, but also in animal feed and fodder with improved characteristics.

The examples of products where RebM disclosedcan be used as a sweetening compound can be as alcoholic beverages such as vodka, wine, beer, liquor, sake, etc; natural juices, refreshing drinks, carbonated soft drinks, diet drinks, zero calorie drinks, reduced calorie drinks and foods, yogurt drinks, instant juices, instant coffee, powdered types of instant beverages, canned products, syrups, fermented soybean paste, soy sauce, vinegar, dressings, mayonnaise, ketchups, curry, soup, instant bouillon, powdered soy sauce, powdered vinegar, types of biscuits, rice biscuit, crackers, bread, chocolates, caramel, candy, chewing gum, jelly, pudding, preserved fruits and vegetables, fresh cream, jam, marmalade, flower paste, powdered milk, ice cream, sorbet, vegetables and fruits packed in bottles, canned and boiled beans, meat and foods boiled in sweetened sauce, agricultural vegetable food products, seafood, ham, sausage, fish ham, fish sausage, fish paste, deep fried fish products, dried seafood products, frozen food products, preserved seaweed, preserved meat, tobacco, medicinal products, and many others. In principal it can have unlimited applications.

The sweetened composition comprises a beverage, non-limiting examples of which include non-carbonated and carbonated beverages such as colas, ginger ales, root beers, ciders, fruit-flavored soft drinks (e.g., citrus-flavored soft drinks such as lemon-lime or orange), powdered soft drinks, and the like; fruit juices originating in fruits or vegetables, fruit juices including squeezed juices or the like, fruit juices containing fruit particles, fruit beverages, fruit juice beverages, beverages containing fruit juices, beverages with fruit flavorings, vegetable juices, juices containing vegetables, and mixed juices containing fruits and vegetables; sport drinks, energy drinks, near water and the like drinks (e.g., water with natural or synthetic flavorants); tea type or favorite type beverages such as coffee, cocoa, black tea, green tea, oolong tea and the like; beverages containing milk components such as milk beverages, coffee containing milk components, cafe au lait, milk tea, fruit milk beverages, drinkable yogurt, lactic acid bacteria beverages or the like; and dairy products.

Generally, the amount of sweetener present in a sweetened composition varies widely depending on the particular type of sweetened composition and its desired sweetness. Those of ordinary skill in the art can readily discern the appropriate amount of sweetener to put in the sweetened composition.

RebM obtained in this disclosure can be used in dry or liquid forms. It can be added before or after heat treatment of food products. The amount of the sweetener depends on the purpose of usage. It can be added alone or in the combination with other compounds.

During the manufacturing of foodstuffs, drinks, pharmaceuticals, cosmetics, table top products, chewing gum the conventional methods such as mixing, kneading, dissolution, pickling, permeation, percolation, sprinkling, atomizing, infusing and other methods can be used.

Thus, compositions disclosedcan be made by any method known to those skilled in the art that provide homogenous even or homogeneous mixtures of the ingredients. These methods include dry blending, spray drying, agglomeration, wet granulation, compaction, co-crystallization and the like.

In solid form RebM disclosed can be provided to consumers in any form suitable for delivery into the comestible to be sweetened, including sachets, packets, bulk bags or boxes, cubes, tablets, mists, or dissolvable strips. The composition can be delivered as a unit dose or in bulk form.

For liquid sweetener systems and compositions convenient ranges of fluid, semi-fluid, paste and cream forms, appropriate packing using appropriate packing material in any shape or form shall be invented which is convenient to carry or dispense or store or transport any combination containing any of the above sweetener products or combination of product produced above.

The composition may include various bulking agents, functional ingredients, colorants, flavors.

A reference herein to a patent document or other matter which is given as prior art is not to be taken as an admission that that document or matter was known or that the information it contains was part of the common general knowledge as at the priority date of any of the claims.

The invention is further illustrated by the following Examples:

### EXAMPLES

### General

Standard genetic techniques, such as overexpression of enzymes in the host cells, as well as for additional genetic modification of host cells, are known methods in the art, such as described in Sambrook and Russel (2001) "Molecular Cloning: A Laboratory Manual (3rd edition), Cold Spring Harbor Laboratory, Cold Spring Harbor Laboratory Press*,* or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

A description of the sequences is set out in Table 1. Sequences described herein may be defined with reference to the sequence listing or with reference to the database accession numbers also set out in Table 1.

### Example 1. Over-expression of ERG20, BTS1 and tHMG in S. cerevisiae

For over-expression of ERG20, BTS1 tHMG1, expression cassettes were designed to be integrated in one locus using technology described in co-pending patent application no. PCT/EP2013/056623. To amplify the 5' and 3' integration flanks for the integration locus, suitable primers and genomic DNA from a CEN.PK yeast strain (van Dijken et al. Enzyme and Microbial Technology 26 (2000) 706-714) was used. The different genes were ordered as cassettes (containing homologous sequence, promoter, gene, terminator, homologous sequence) at DNA2.0. The genes in these cassettes were flanked by constitutive promoters and terminators. See Table 2. Plasmid DNA from DNA2.0 containing the ERG20, tHMG1 and BTS1 cassettes were dissolved to a concentration of 100 ng/µl. In a 50 µl PCR mix 20 ng template was used together with 20 pmol of the primers. The material was dissolved to a concentration of 0.5 µg/µl.

**Table 2: Composition of the over-expression constructs.**

| ***Promoter*** | ***ORF*** | ***Terminator*** |
|---|---|---|
| Eno2 (SEQ ID NO: 201) | Erg20 (SEQ ID NO: 81) | Adhl (SEQ ID NO: 212) |
| Fba1 (SEQ ID NO: 202) | tHMG1 (SEQ ID NO: 79) | Adh2 (SEQ ID NO: 213) |
| Tef1 (SEQ ID NO: 203) | Btsl (SEQ ID NO:83) | Gmp1 (SEQ ID NO: 214) |

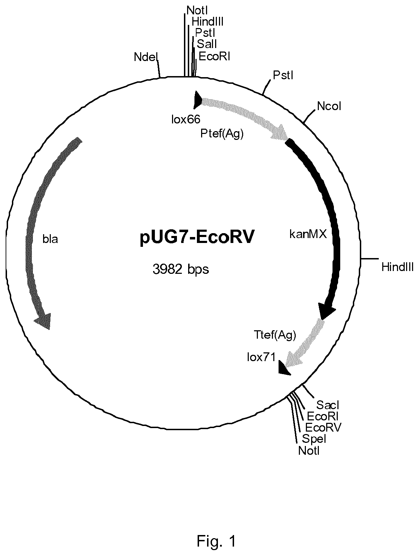

For amplification of the selection marker, the pUG7-EcoRV construct (Figure 1) and suitable primers were used. The KanMX fragment was purified from gel using the Zymoclean Gel DNA Recovery kit (ZymoResearch). Yeast strain Cen.PK113-3C was transformed with the fragments listed in Table 3.

**Table 3: DNA fragments used for transformation of ERG20, tHMG1 and BTS1**

| ***Fragment*** |
|---|
| 5'YPRcTau3 |
| ERG20 cassette |
| tHMG1 cassette |
| KanMX cassatte |
| BTS1 cassette |
| 3'YPRcTau3 |

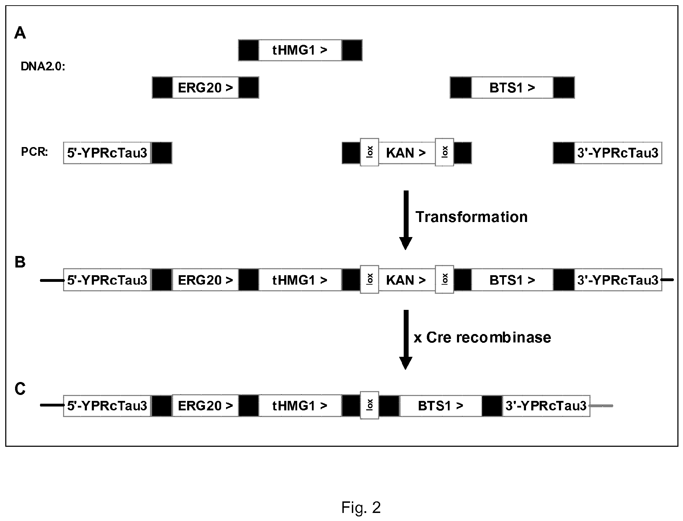

After transformation and recovery for 2.5 hours in YEPhD (yeast extract phytone peptone glucose; BBL Phytone Peptone from BD) at 30°C the cells were plated on YEPhD agar with 200 µg/ml G418 (Sigma). The plates were incubated at 30°C for 4 days. Correct integration was established with diagnostic PCR and sequencing. Over-expression was confirmed with LC/MS on the proteins. The schematic of the assembly of ERG20, tHMG1 and BTS1 is illustrated in Figure 2. This strain is named STV002.

Expression of the CRE-recombinase in this strain led to out-recombination of the KanMX marker. Correct out-recombination, and presence of ERG20, tHMG and BTS1 was established with diagnostic PCR.

### Example 2. Knock down of Erg9

For reducing the expression of Erg9, an Erg9 knock down construct was designed and used that contains a modified 3' end, that continues into the TRP1 promoter driving TRP1 expression.

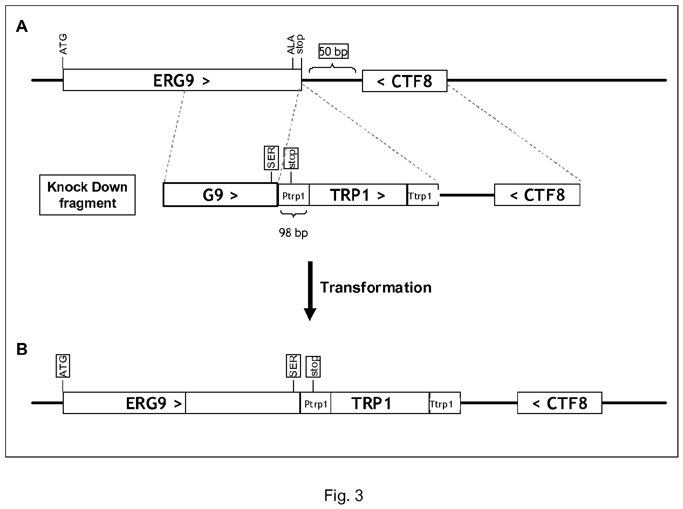

The construct containing the Erg9-KD fragment was transformed to E. *coli* TOP10 cells. Transformants were grown in 2PY(2 times Phytone peptone Yeast extract), sAMP medium. Plasmid DNA was isolated with the QIAprep Spin Miniprep kit (Qiagen) and digested with Sall-HF (New England Biolabs). To concentrate, the DNA was precipitated with ethanol. The fragment was transformed to *S*. *cerevisiae,* and colonies were plated on mineral medium (Verduyn et al, 1992. Yeast 8:501-517) agar plates without tryptophan. Correct integration of the Erg9-KD construct was confirmed with diagnostic PCR and sequencing. The schematic of performed transformation of the Erg9-KD construct is illustrated in Figure 3. The strain was named STV003.

### Example 3. Over-expression of UGT2 1a

For over-expression of UGT2_1a, technology was used as described in co-pending patent application nos. PCT/EP2013/056623 and PCT/EP2013/055047. The UGT2_1a was ordered as a cassette (containing homologous sequence, promoter, gene, terminator, homologous sequence) at DNA2.0. For details, see Table 4. To obtain the fragments containing the marker and Cre-recombinase, technology was used as described in co-pending patent application no. PCT/EP2013/055047. The NAT marker, conferring resistance to nourseothricin was used for selection.

**Table 4: Composition of the over-expression construct**

| ***Promoter*** | ***ORF*** | ***Terminator*** |
|---|---|---|
| Pgk1 (SEQ ID NO: 204) | UGT2_1a (SEQ ID NO: 87) | Adh2 (SEQ ID NO: 213) |

Suitable primers were used for amplification. To amplify the 5' and 3' integration flanks for the integration locus, suitable primers and genomic DNA from a CEN.PK yeast strain was used.

*S*. *cerevisiae* yeast strain STV003 was transformed with the fragments listed in Table 5, and the transformation mix was plated on YEPhD agar plates containing 50 µg/ml nourseothricin (Lexy NTC from Jena Bioscience).

**Table 5: DNA fragments used for transformation of UGT2_1a**

| ***Fragment*** |
|---|
| 5'Chr09.01 |
| UGT2_1a cassette |
| NAT-CR |
| RE |
| 3'Chr09.01 |

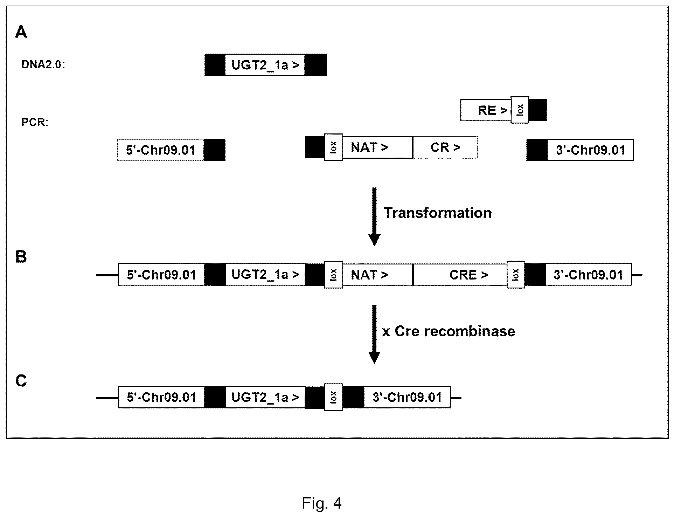

Expression of the CRE recombinase is activated by the presence of galactose. To induce the expression of the CRE recombinase, transformants were restreaked on YEPh Galactose medium. This resulted in out-recombination of the marker(s) located between lox sites. Correct integration of the UGT2a and out-recombination of the NAT marker was confirmed with diagnostic PCR. The resulting strain was named STV004. The schematic of the performed transformation of the UGT2_1 a construct is illustrated in Figure 4.

### Example 4. Over-expression of production pathway to RebA: CPS. KS, KO. KAH, CPR, UGT1, UGT3 and UGT4.

All pathway genes leading to the production of RebA were designed to be integrated in one locus using technology described in co-pending patent application no. PCT/EP2013/056623. To amplify the 5' and 3' integration flanks for the integration locus, suitable primers and genomic DNA from a CEN.PK yeast strain was used. The different genes were ordered as cassettes (containing homologous sequence, promoter, gene, terminator, homologous sequence) at DNA2.0 (see Table 5 for overview). The DNA from DNA2.0 was dissolved to 100 ng/µl. This stock solution was further diluted to 5 ng/µl, of which 1 µl was used in a 50µl-PCR mixture. The reaction contained 25 pmol of each primer. After amplification, DNA was purified with the NucleoSpin 96 PCR Clean-up kit (Macherey-Nagel) or alternatively concentrated using ethanol precipitation.

**Table 6. Sequences used for production pathway to RebA**

| ***Promoter*** | ***ORF*** | ***SEQ ID*** | ***Terminator*** |
|---|---|---|---|
| Kl prom 12.pro (SEQ ID NO: 205) | trCPS_SR | 61 | Sc ADH2.ter(SEQ ID NO:) |
| Sc PGK1.pro (SEQ ID NO: 204) | trKS_SR | 65 | Sc TALl.ter (SEQ ID NO: 215) |
| Sc ENO2.pro (SEQ ID NO: 201) | KO_2 | 23 | Sc TPI1.ter (SEQ ID NO: 216) |
| Ag lox TEFl.pro (SEQ ID NO:206 ) | KANMX | 211 | Ag TEF1_lox.ter (SEQ ID NO: 217) |
| Sc TEFl.pro (SEQ ID NO: 203) | KAH_4 | 33 | Sc GPM1.ter (SEQ ID NO: 214) |
| Kl prom 6.pro (SEQ ID NO: 207) | CPR_SR | 59 | Sc PDCl.ter (SEQ ID NO: 218) |
| Kl prom 3.pro (SEQ ID NO: 221) | UGT1_SR | 71 | Sc TDH1.ter (SEQ ID NO: 219) |
| Kl prom 2.pro (SEQ ID NO: 222) | UGT3_SR | 73 | Sc ADH1.ter (SEQ ID NO: 212) |
| Sc FBAl.pro (SEQ ID NO: 202) | UGT4_SR | 75 | Sc ENO1.ter (SEQ ID NO: 220) |

All fragments for the pathway to RebA, the marker and the flanks (see overview in Table 7) were transformed to *S*. *cerevisiae* yeast strain STV004. After overnight recovery in YEPhD at 20°C the transformation mixes were plated on YEPhD agar containing 200 µg/ml G418. These were incubated 3 days at 25°C and one night at RT.

**Table 7. DNA fragments used for transformation of CPS, KS, KO, KanMX, KAH, CPR, UGT1, UGT3 and UGT4.**

| ***Fragment*** |
|---|
| 5'INT1 |
| CPS cassette |
| KS cassette |
| KO cassette |
| KanMX cassette |
| KAH cassette |
| CPR cassette |
| UGT1 cassette |
| UGT3 cassette |
| UGT4 cassette |
| 3'INT1 |

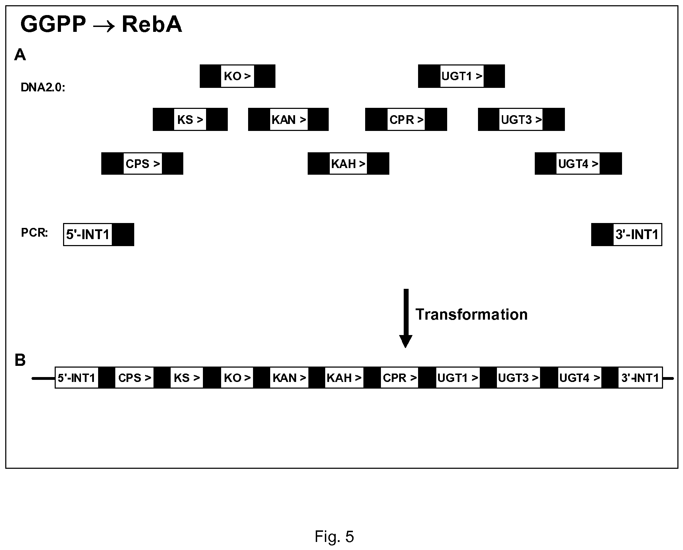

Correct integration was confirmed with diagnostic PCR and sequence analysis (3500 Genetic Analyzer, Applied Biosystems). The sequence reactions were done with the BigDye Terminator v3.1 Cycle Sequencing kit (Life Technologies). Each reaction (10 µl) contained 50 ng template and 3.2 pmol primer. The products were purified by ethanol/EDTA precipitation, dissolved in 10 µl HiDi formamide and applied onto the apparatus. The strain was named STV016. The schematic of how the pathway from GGPP to RebA is integrated into the genome is illustrated in Figure 5.

### Example 5: Construction of strain STV027

To remove the KanMX marker from the chromosome of strain STV016, this strain was transformed with plasmid pSH65, expressing Cre-recombinase (Güldender, 2002). Subsequently plasmid pSH65 was cured from the strain by growing on non-selective medium (YEP 2% glucose). The resulting, KanMX-free and pSH65-free strains, as determined by plating on plates containing 200 µg G418/ml or 20 µg phleomycin/ml, where no growth should occur, was named STV027. Absence of the KanMX marker was furthermore confirmed with diagnostic PCR.

### Example 6. RebM production by Saccharomyces cerevisiae

Although the STV027 strain was initially designed for production of RebA, in this Example the strain is shown to produce the steviol glycoside, RebM.

### 6.1 Construction of recombinant host for diterpenes or glycosylated diterpenes production

The construction of the recombinant host for the production of RebM, STV027, is described above in Example 5.

### 6.2 Detection of rebaudioside M by LC and MS

The purified fraction (see 6.4 below) was analyzed with a LTQ orbitrap (Thermo), equipped with a Acella LC and a Waters Acquity UPLC BEH amide 1.7µm 2.1*150 mm column. Eluentia used for the separation were A: 10 mM Ammonium acetate in MilliQ water, B: Acetonitrile, and the gradient started at 65 % A and was kept here for 1.5 minutes, then increased to 95 % B in 0.5 minutes and kept here for 0.5 minutes before regeneration for 1.5 min at 65 % A. The flow-rate was 0.6 ml/min and the column temperature was kept at 50 C. Mass spectral analysis was performed in electrospray negative ionization mode, scanning from m/z 100-1800 at a resolution of 7500.

### 6.3 Diterpene or glycosylated diterpene fermentation

The yeast strain STV027 constructed as described above, was cultivated in shake-flask (500ml with 50 ml medium) for 2 days at 30°C and 280 rpm. The medium was based on Verduyn et al. (Verduyn C, Postma E, Scheffers WA, Van Dijken JP. Yeast, 1992 Jul;8(7):501-517), with modifications in the carbon and nitrogen sources, as described in Table 8.

**Table 8. Preculture medium composition**

| **Raw material** | **Formula** | **Concentration (g/kg)** |
|---|---|---|
| Galactose | C₆H₁₂O₆ | 20.0 |
| Urea | (NH₂)₂CO | 2.3 |
| Potassium dihydrogen phosphate | KH₂PO₄ | 3.0 |
| Magnesium sulphate | MgSO₄ . 7H₂O | 0.5 |
| Trace element solution | | 1 |
| Vitamin solution | | 1 |

| **Component** | **Formula** | **Concentration (g/kg)** |
|---|---|---|
| EDTA | C₁₀H₁₄N₂Na₂O₈. 2H₂O | 15.00 |
| Zincsulphate . 7H₂O | ZnSO₄.7H₂O | 4.50 |
| Manganesechloride . 2H₂O | MnCl₂ . 2H₂O | 0.84 |
| Cobalt (II) chloride . 6H₂O | CoCl₂ . 6H₂O | 0.30 |
| Cupper (II) sulphate . 5H₂O | CuSO₄ . 5H₂O | 0.30 |
| Sodium molybdenum . 2H₂O | Na₂MoO₄ . 2H₂O | 0.40 |
| Calciumchloride . 2H₂O | CaCl₂. 2H₂O | 4.50 |
| Ironsulphate . 7H₂O | FeSO₄.7H₂O | 3.00 |
| Boric acid | H₃BO₃ | 1.00 |
| Potassium iodide | KI | 0.10 |
| Biotin (D-) | C₁₀H₁₆N₂O₃S | 0.05 |
| Ca D(+) panthothenate | C₁₈H₃₂CaN₂O₁₀ | 1.00 |
| Nicotinic acid | C₆H₅NO₂ | 1.00 |
| Myo-inositol | C₆H₁₂O₆ | 25.00 |
| Thiamine chloride hydrochloride | C₁₂H₁₈Cl₂N₄OS . xH₂O | 1.00 |
| Pyridoxol hydrochloride | C₈H₁₂CINO₃ | 1.00 |
| p-aminobenzoic acid | C₇H₇NO₂ | 0.20 |

| | | |
|---|---|---|
| ^{a}Trace elements solution ^{b}Vitamin solution | | |

Subsequently, 6ml of the content of the shake-flask was transferred into a fermenter (starting volume 0.3 L), which contained the medium as set out in Table 9.

**Table 9. Composition fermentation medium**

| **Raw material** | | **Final Concentration (g/kg)** |
|---|---|---|
| Ammonium sulphate | (NH₄)₂SO₄ | 1 |
| Potassium dihydrogen phosphate | KH₂PO₄ | 10 |
| Magnesium sulphate | MgSO₄ . 7H₂O | 5 |
| Trace element solution | - | 8 |
| Vitamin solution | - | 8 |

The pH was controlled at 5.0 by addition of ammonia (12.5 wt%). Temperature was controlled at 27°C. pO₂ was controlled at 40% by adjusting the stirrer speed. Glucose concentration was kept limited by controlled feed to the fermenter.

**Table 10. Composition of the fermentation feed medium**

| **Raw material** | **Formula** | **Final Concentrate (g/kg)** |
|---|---|---|
| Glucose.1aq | C₆H₁₂O₆.1 aq | 330 |
| Potassium dihydrogen phosphate | KH₂PO₄ | 10 |
| Magnesium sulphate heptahydrate | MgSO₄.7H₂O | 5 |
| Verduyn trace elements solution | | 8 |
| Verduyn vitamin solution | | 8 |

### 6.4 Rebaudioside M production in the fermentation broth during fed-batch fermentation

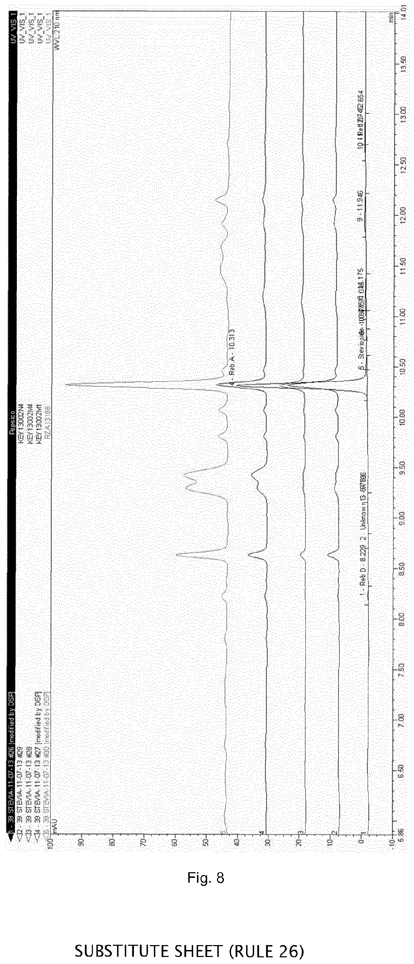

Fermentation broth was heat shocked at 70-90 °C to kill the yeast cells and make them open. The heat shocked broth was spray dried. Dried biomass was extracted twice with 90 % ethanol at 50-60 °C. The extracts were combined and evaporated to about 1/10 - 1/30 of their original volume. The evaporated extract was diluted with water to reach the ethanol concentration 20 %. The analytical HPLC chromatogram of the extract is the top curve in Figure 8. A voluminous precipitate formed on dilution of extract with water was removed by centrifugation. The analytical HPLC chromatogram of the centrifugate is the next top curve in Figure 8. The 20 % ethanol feed with pH around 4.5 was applied on a column packed with DIAION® HP20 and eluted with gradient 14CV 20-80% ethanol. The analytical HPLC chromatogram of the first eluate is the third from the top curve in Figure 8. pH in the pooled fraction was adjusted to 8.5 and it was applied again on a column packed with DIAION® HP20 and eluted stepwise with 4 CV of 80 % ethanol. The analytical HPLC chromatogram of the second eluate is the fourth curve from the top curve in Figure 8. The peak in analytical chromatograms eluting at 8.7 min is eluting at 8.7 min is RebM.

The presence of RebM was confirmed by LC and MS carried out on the first eluate (as described in the preceding paragraph) using the conditions set out at 6.2 above. Reb M elutes at tr=0.72 min, just after reb D at tr=0.63. Reb M is characterized by a deprotonated molecule of m/z 1289.5286. The elemental composition could be estimated using accurate mass analysis.

### Example 7. Description of the construction of STV2019

Two *Yarrowia lipolytica* strains of mating types MATA and MATB were engineered for steviol glycoside production. These strains were mated, the diploid sporulated, and spores with steviol glycoside production were selected. One of these spores was further developed for the production of steviol glycosides, including the production of rebaudioside M.

### Example 7.11. Description of steviol glycoside production strain ML14094 (MAT-A lineage)

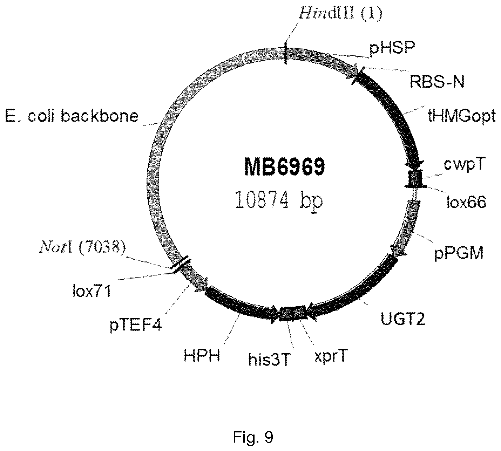

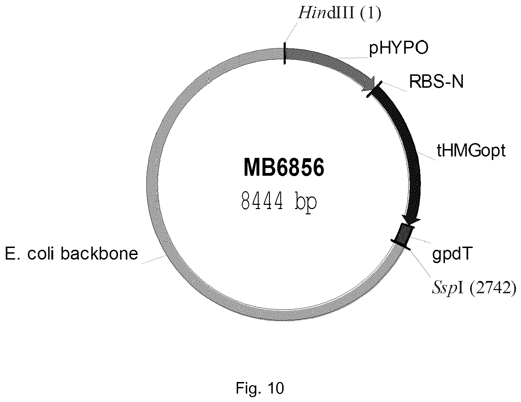

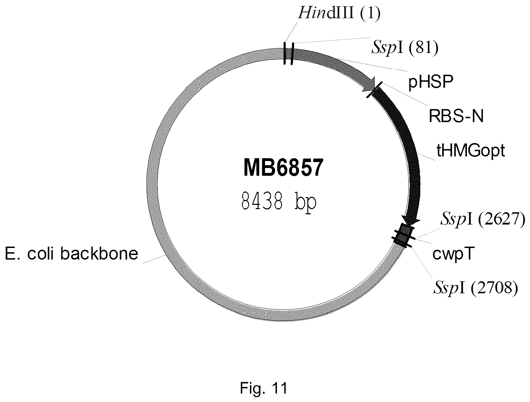

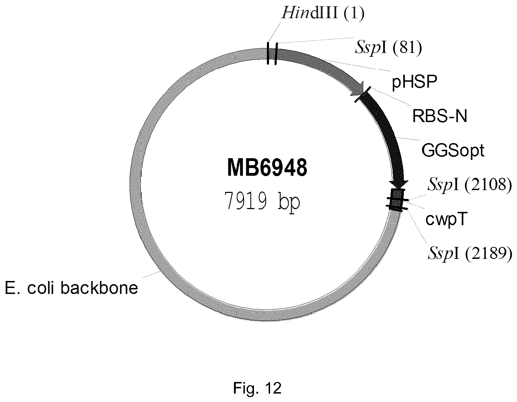

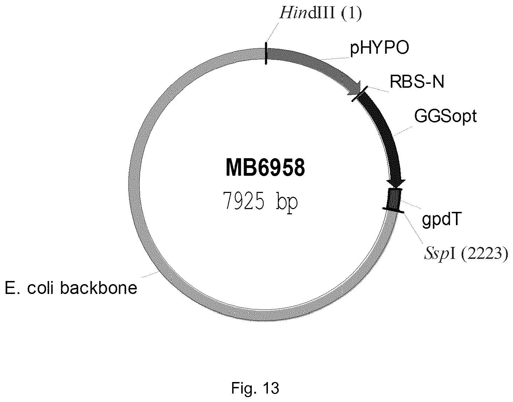

Step 1. Strain ML10371 (MAT-A, lys1-, ura3-, leu2-) was transformed with 5 defined DNA fragments. All transformations were carried out via a lithium acetate/PEG fungal transformation protocol method and transformants were selected on minimal medium, YPD + 100 ug/ml nourseothricin or YPD + 100 ug/ml hygromycin, as appropriate.
1) a 7.0 kb DNA fragment isolated by gel purification following HindIII/NotI digestion of plasmid MB6969 (Figure 9). This construct encodes a synthetic construct for the overexpression of UGT2 (SEQ ID NO: 242) linked to the pPGM promoter (SEQ ID NO: 258) and xprT terminator (SEQ ID NO: 269) and the HPH hygromycin resistance gene (SEQ ID NO: 245), together flanked by lox sites (SEQ ID NOs: 232 and 233), and a synthetic construct for the overexpression of the codon optimized *Y. lipolytica* hydroxymethylglutaryl-coenzyme A reductase open reading frame lacking the 5' membrane anchor sequence (tHMGopt: SEQ ID NO: 234) linked to the pHSP promoter (SEQ ID NO: 253) and cwpT terminator (SEQ ID NO: 265).
2) a 2.7 kb DNA fragment isolated by gel purification following Hindlll/Sspl digestion of MB6856 (Figure 10). This construct encodes tHMGopt linked to the pHYPO (SEQ ID NO: 254) promoter and gpdT terminator (SEQ ID NO: SEQ ID NO: 266).
3) a 2.5 kb DNA fragment isolated by gel purification following Sspl digestion of MB6857 (Figure 11). This construct encodes tHMGopt linked to the pHSP promoter and cwpT terminator.
4) a 2.0 kb DNA fragment isolated by gel purification following Sspl digestion of MB6948 (Figure 12). This construct encodes a synthetic construct for the overexpression of the codon optimized *Y. lipolytica* geranyl-geranyl-pyrophosphate synthetase (GGSopt: SEQ ID NO: 235) linked to the pHSP promoter and cwpT terminator.
5) a 2.2 kb DNA fragment isolated by gel purification following Hindlll/Sspl digestion of MB6958 (Figure 13).

This construct encodes GGSopt linked to the pHYPO promoter and gpdT terminator. The resulting strain was denoted ML13462.

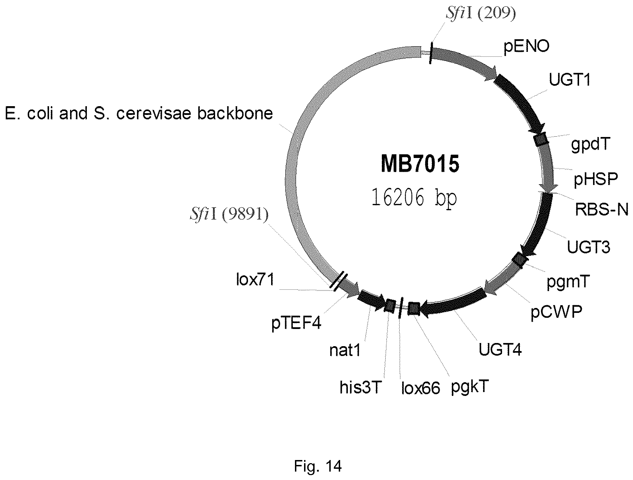

Step 2. Strain ML13462 was transformed with a 9.7 kb fragment isolated by gel purification following Sfil digestion of plasmid MB7015 (Figure 14). This construct encodes a synthetic construct for the overexpression of UGT1 (SEQ ID NO: 241) linked to the pENO promoter (SEQ ID NO: 255) and gpdT terminator (SEQ ID NO: 272), UGT3 (SEQ ID NO: 243) linked to the pHSP promoter and pgmT (SEQ ID NO: 267) terminator, UGT4 (SEQ ID NO: 244) linked to the pCWP promoter (SEQ ID NO: 257) and pgkT terminator (SEQ ID NO: 268), and the lox-flanked nourseothricin resistance marker (NAT: SEQ ID NO: 246). Note that placement of lox sites allows for subsequent removal of nourseothricin resistance via CRE recombinase mediated recombination. A nourseothricin resistant isolate was denoted ML13500.

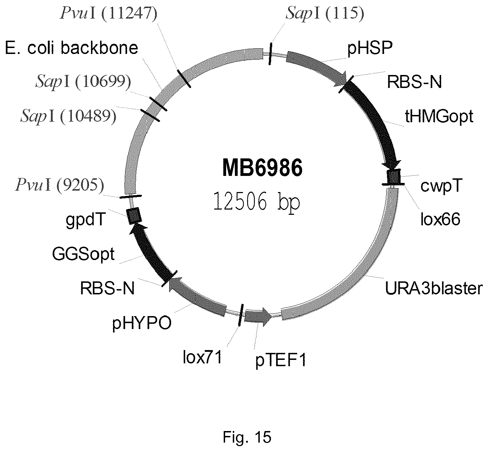

Step 3. Strain ML13500 was transformed with a 9.1 kb fragment isolated by gel purification following Pvul/Sapl digestion of plasmid MB6986 (Figure 15). This construct encodes tHMGopt linked to the pHSP promoter and cwpT terminator, the lox-flanked URA3blaster prototrophic marker (SEQ ID NO: 252), and GGSopt linked to the pHYPO promoter and gpdT terminator (SEQ ID NO: 272). Transformants were selected on minimal medium lacking uracil. One selected uracil prototroph was denoted ML13723.

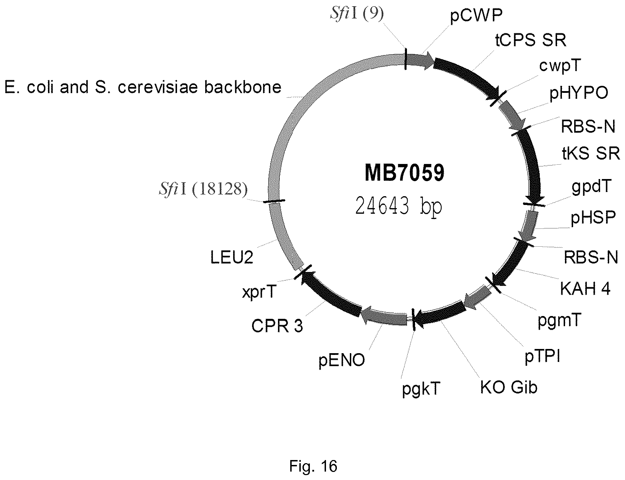

Step 4. Strain ML13723 was transformed with an 18.1 kb fragment isolated by gel purification following Sfil digestion of plasmid MB7059 (Figure 16). MB7059 encodes the tCPS_SR (SEQ ID NO: 236) linked to pCWP promoter and cwpT terminator, the tKS_SR (SEQ ID NO: 237) linked to the pHYPO promoter and gpdT terminator, the KAH_4 (SEQ ID NO: 239) linked to the pHSP promoter and pgmT terminator (SEQ ID NO: 273), the KO_Gib (SEQ ID NO: 238) linked to the pTPI promoter (SEQ ID NO: 256) and pgkT terminator (SEQ ID NO: 274), the CPR_3 (SEQ ID NO: 240) linked to the pENO promoter and xprT terminator and the native *Y. lipolytica* LEU2 locus (SEQ ID NO: 250). One selected rebaudioside A-producing transformant was denoted ML14032.

Step 5. Strain ML14032 was struck to YPD and grown overnight and then struck to 5-FOA plates to allow for recombination mediated loss of the URA3 marker introduced in Step 3. One selected 5-FOA resistant transformant was denoted ML14093.

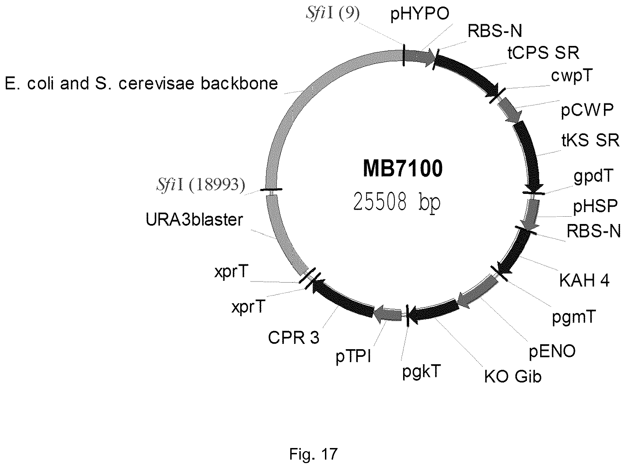

Step 6. Strain ML14093 was transformed with a 19.0 kb fragment isolated by gel purification following Sfil digestion of plasmid MB7100 (Figure 17). MB7100 encodes the tCPS_SR linked to the pHYPO promoter and cwpT terminator, the tKS_SR (SEQ ID NO: 237) linked to the pCWP promoter and gpdT terminator, the KAH_4 linked to the pHSP promoter and pgmT terminator, the KO_Gib linked to the pENO promoter and pgkT terminator, the CPR_3 linked to the pTPI promoter and xprT terminator and URA3blaster prototrophic marker. Transformants were selected on minimal medium lacking uracil. One selected rebaudioside A producing uracil prototroph was denoted ML14094.

### Example 7.2. Description of steviol glycoside production strain ML14087 (MAT-B lineage)

Step 1. Strain ML13206 (MAT-B, ade1-, ure2-, leu2-) was transformed with 5 defined DNA fragments. All transformations were carried out via a lithium acetate/PEG fungal transformation protocol method and transformants were selected on minimal medium, YPD + 100 ug/ml nourseothricin or YPD + 100 ug/ml hygromycin, as appropriate.
1) a 7.0 kb DNA fragment isolated by gel purification following HindIII/NotI digestion of plasmid MB6969 (Figure 9). This construct encodes a synthetic construct for the overexpression of the codon pair optimized (CpO) ORF of UGT2 linked to the pPGM promoter and xprT terminator and the HPH hygromycin resistance gene, together flanked by lox sites, and a synthetic construct for the overexpression of the codon optimized Y. lipolytica hydroxymethylglutaryl-coenzyme A reductase open reading frame lacking the 5' membrane anchor sequence (tHMGopt) linked to the pHSP promoter and cwpT terminator.
2) a 2.7 kb DNA fragment isolated by gel purification following Hindlll/Sspl digestion of MB6856 (Figure 10). This construct encodes tHMGopt linked to the pHYPO promoter and gpdT terminator.
3) a 2.5 kb DNA fragment isolated by gel purification following Sspl digestion of MB6857 (Figure 11). This construct encodes tHMGopt linked to the pHSP promoter and cwpT terminator.
4) a 2.0 kb DNA fragment isolated by gel purification following Sspl digestion of MB6948 (Figure 12). This construct encodes a synthetic construct for the overexpression of the codon optimized Y. lipolytica geranyl-geranyl-pyrophosphate synthetase (GGSopt) linked to the pHSP promoter and cwpT terminator.
5) a 2.2 kb DNA fragment isolated by gel purification following Hindlll/Sspl digestion of MB6958 (Figure 13). This construct encodes GGSopt linked to the pHYPO promoter and gpdT terminator.

The resulting strain was denoted ML13465.

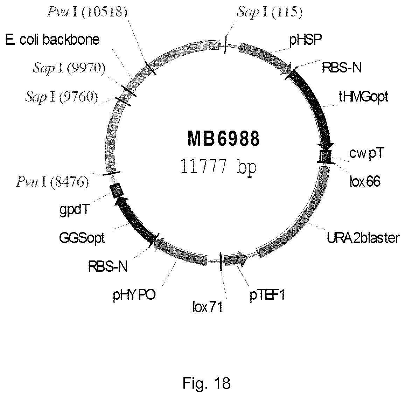

Step 2. Strain ML13465 was transformed with 2 defined DNA fragments:
1). a 9.7 kb fragment isolated by gel purification following Sfil digestion of plasmid MB7015 (Figure 14). This construct encodes a synthetic construct for the overexpression of UGT1 linked to the pENO promoter and gpdT terminator, UGT3 linked to the pHSP promoter and pgmT terminator, UGT4 (SEQ ID NO: 244) linked to the pCWP promoter and pgkT terminator, and the lox-flanked nourseothricin resistance marker (NAT). Note that placement of lox sites allows for subsequent removal of nourseothricin resistance via CRE recombinase mediated recombination.
2). a 9.1 kb fragment isolated by gel purification following Pvul/Sapl digestion of plasmid MB6988 (Figure 18). This construct encodes tHMGopt linked to the pHSP promoter and cwpT terminator, the lox-flanked URA2blaster prototrophic marker (SEQ ID NO: 251), and GGSopt linked to the pHYPO promoter and gpdT terminator. Strains were selected on YPD + 100 ug/ml nourseothricin and replica plated to minimal medium lacking uracil. A nourseothricin resistant, uracil prototrophic isolate was denoted ML13490

Step 3. Strain ML13490 was struck to YPD and grown overnight and then struck to 5-FOA plates to allow for recombination mediated loss of the URA2 marker introduced in step 3 above. One selected 5-FOA resistant transformant was denoted ML13501.

Step 4. Strain ML13501 was transformed with a 9.1 kb fragment isolated by gel purification following Pvul/Sapl digestion of plasmid MB6988 (Figure 18). Transformants were selected on minimal medium lacking uracil. One selected uracil prototroph was denoted ML13724.

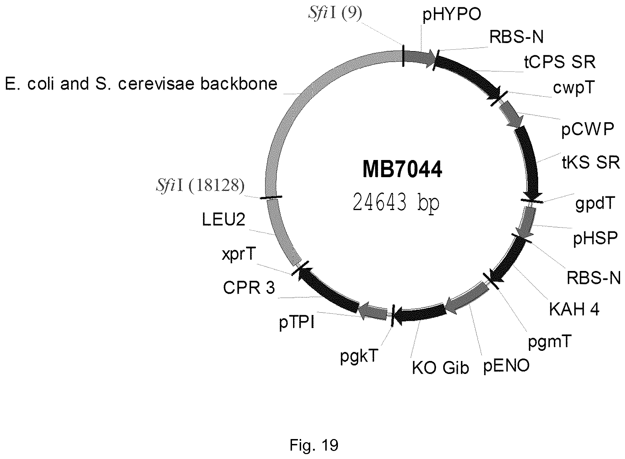

Step 5. Strain ML13724 was transformed with an 18.1 kb fragment isolated by gel purification following Sfil digestion of plasmid MB7044 (Figure 19). MB7044 encodes the tCPS_SR linked to the pHYPO promoter and cwpT terminator, the tKS_SR linked to the pCWP promoter and gpdT terminator, the KAH_4 linked to the pHSP promoter and pgmT terminator, the KO_Gib linked to the pENO promoter and pgkT terminator, the CPR_3 linked to the pTPI promoter and xprT terminator and the LEU2 locus. One selected rebaudioside A-producing transformant was denoted ML14044.

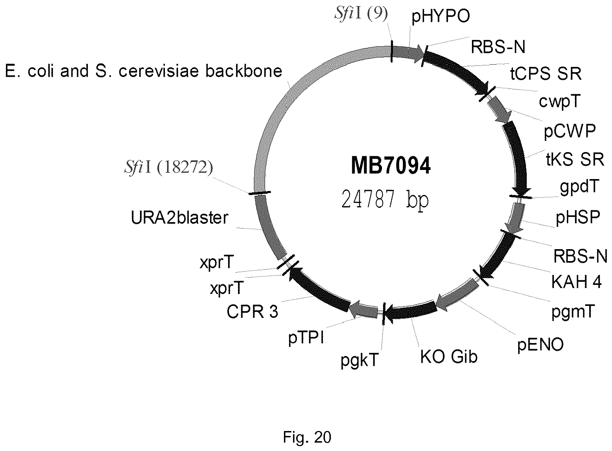

Step 6. Strain ML14044 was struck to YPD and grown overnight and then struck to 5-FOA plates to allow for recombination mediated loss of the URA2 marker introduced in Step 4 above. One selected 5'-FOA resistant transformant was denoted ML14076. Step 7. Strain ML14076 was transformed with a 19.0 kb fragment isolated by gel purification following Sfil digestion of plasmid MB7094 (Figure 20). MB7094 encodes the tCPS_SR linked to the pHYPO promoter and cwpT terminator, the tKS_SR linked to the pCWP promoter and gpdT terminator, the KAH_4 linked to the pHSP promoter and pgmT terminator, the KO_Gib linked to the pENO promoter and pgkT terminator, the CPR_3 linked to the pTPI promoter and xprT terminator and URA2blaster prototrophic marker. Transformants were selected on minimal medium lacking uracil. One selected rebaudioside A producing uracil prototroph was denoted ML14087.

### Example 7.3. Mating MATA and MATB lineage and selecting steviol glycoside-producing progeny

Strains of opposite mating types (ML14094 and ML14087) with complementary nutritional deficiencies (ADE1+ lys1- and ade1- LYS1+) were allowed to mate and then plated on selective media that would allow only diploids to grow (minimal media lacking both adenine and lysine). Diploid cells (ML14143) were then induced to undergo meiosis and sporulation by starvation, and the resulting haploid progenies were replica-plated to identify prototrophic isolates with hygromycin and nourseothricin resistance. One selected rebaudioside A-producing strain was denoted STV2003.

### Example 7.4. Increasing steviol glycoside production by over-expression of CPS, KAH4, UGT2 and UGT4.

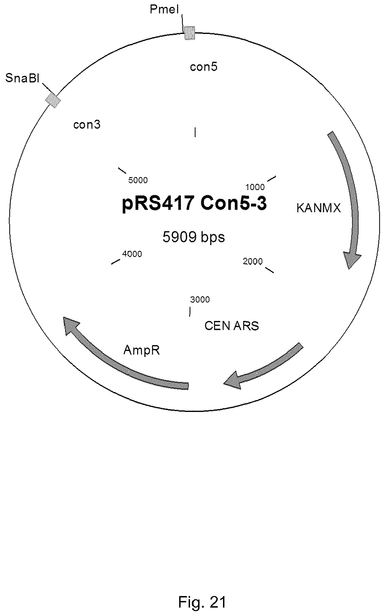

Additional copies of CPS, KAH, UGT2 and UGT4 were transformed to STV2003, and integrated in the GSY1 (YALI0F18502) locus. The GSY1 locus is thereby disrupted. To amplify the 5' and 3' integration flanks for the GSY1 integration locus, suitable primers and genomic DNA from Yarrowia strain ML326 was used (SEQ ID NOs: 224 to 227). These flanks contain connector sequences (5 and a and f and 3) for proper assembly in the pRS417_3_5 vector (Figure 21) together with the cassettes (described below). The heterologous genes were ordered as cassettes (containing connector sequence, promoter, gene, terminator, connector sequence) at DNA2.0, or assembled in house. After amplification, DNA was purified with the NucleoSpin 96 PCR Clean-up kit (Macherey-Nagel).

**Table 11. Cassettes used for transformation to STV2003**

| ***connector*** | **Promoter** | ***ORF*** | ***SEQ ID*** | ***Terminator*** | ***connector*** |
|---|---|---|---|---|---|
| a | SCP2p (SEQ ID NO: 260) | CPS | 236 | gpdT (SEQ ID NO: 266) | b |
| b | ENO1p (SEQ ID NO: 255) | KAH | 239 | pgmT (SEQ ID NO: 267) | c |
| c | Ag_lox_TEF1.pro (SEQ ID NO: 264) | KanMX | 247 | Ag_TEF1_lox TPI1.ter (SEQ ID NO: 275) | d |
| d | HSPp (SEQ ID NO: 253) | UGT4 | 244 | pgkT (SEQ ID NO: 268) | e |
| e | YP005p (SEQ ID NO: 259) | UGT2 | 242 | xprT (SEQ ID NO: 269) | f |

All cassettes, flanks and the linearized pRS417 5_3 vector (SnaBI/Pmel, Figure M) were transformed to *S*. *cerevisiae* strain CEN.PK114-7D. Transformation mixes were plated on YEPhD agar containing 200 µg/ml G418, and incubated 4 days at 30°C. A select number of correct transformants were grown in YEPhD liquid medium. From these cultures plasmid DNA was isolated using the the Qiaprep Spin Miniprep Kit (Qiagen, 27106) according to suppliers'instruction.

The isolated plasmid was transformed to chemically competent 10-Beta E. coli cells (NEB, C3019H) according to suppliers' instructions. The transformed cells were allowed to recovered for 1 hour in 1 ml SOC at 37°C 250 rpm. An aliquot was plated on 2xPY + Amp and incubated overnight at 37°C. Single colonies were selected from the transformation plate and used to inoculate 2xPY + Amp, and incubated at 30°C with shaking at 250 rpm.

Plasmid DNA was isolated from the *E. coli* clones using the NucleoSpin Plasmid Kit (Machery Nagel, REF 740588.250) according to suppliers' instructions. Diagnostic PCR was performed to confirm proper plasmid. The plasmid DNA isolated was used as template for amplification of the complete assembly. Two fragments were amplified, with overhang in the KanMX marker with primers DBC-05793 (SEQ ID NO:28) and DBC-10726 (SEQ ID NO: 229) for amplification of the CPS, KAH and part of the KanMX gene, and primers DBC-10727 (SEQ ID NO: 230) and DBC-05816 (SEQ ID NO: 231) for the amplification of part of the KanMX gene and UGT4 and UGT2.

After amplification, both fragments were purified using the Nucleospin Gel and PCR Clean-up (Machery Nagel, REF740609.250) according to suppliers' instructions. The DNA was then transformed to *Y. lipolytica* yeast strain STV2003. The transformation mixes were plated on YEPhD agar containing 200 µg/ml G418 and incubated 3 days at 30°C. Tranformants were checked with diagnostic PCR for correct integration. One correct transformant was named STV2019.

### Example 7.5. Production of RebM with strain STV2019

The *Y. lipolytica* strains STV2019 constructed as described above, were cultivated in shake-flask (0.5 l with 50 ml medium) for 2 days at 30°C and 280 rpm. The medium was based on Verduyn et al. (Verduyn C, Postma E, Scheffers WA, Van Dijken JP. Yeast, 1992 Jul;8(7):501-517), with modifications in the carbon and nitrogen sources, as described in Table 12.

**Table 12. Preculture medium composition**

| **Raw material** | **Formula** | **Concentration (g/kg)** |
|---|---|---|
| Glucose.1aq | C₆H₁₂O₆.1H₂O | 66 |
| Urea | (NH₂)₂CO | 6.9 |
| Potassium dihydrogen phosphate | KH₂PO₄ | 9.0 |
| Magnesium sulphate | MgSO₄ . 7H₂O | 1.5 |
| Trace element solution | | 3 |
| Vitamin solution | | 3 |

| **Component** | **Formula** | **Concentration (g/kg)** |
|---|---|---|
| EDTA | C₁₀H₁₄N₂Na₂O₈. 2H₂O | 15.00 |
| Zincsulphate . 7H₂O | ZnSO₄.7H₂O | 4.50 |
| Manganesechloride . 2H₂O | MnCl₂ . 2H₂O | 0.84 |
| Cobalt (II) chloride . 6H₂O | CoCl₂ . 6H₂O | 0.30 |
| Cupper (II) sulphate . 5H₂O | CuSO₄ . 5H₂O | 0.30 |
| Sodium molybdenum . 2H₂O | Na₂MoO₄ . 2H₂O | 0.40 |
| Calciumchloride . 2H₂O | CaCl₂. 2H₂O | 4.50 |
| Ironsulphate . 7H₂O | FeSO₄.7H₂O | 3.00 |
| Boric acid | H₃BO₃ | 1.00 |
| Potassium iodide | KI | 0.10 |
| Biotin (D-) | C₁₀H₁₆N₂O₃S | 0.05 |
| Ca D(+) panthothenate | C₁₈H₃₂CaN₂O₁₀ | 1.00 |
| Nicotinic acid | C₆H₅NO₂ | 1.00 |
| Myo-inositol | C₆H₁₂O₆ | 25.00 |
| Thiamine chloride hydrochloride | C₁₂H₁₈Cl₂N₄OS . xH₂O | 1.00 |
| Pyridoxol hydrochloride | C₈H₁₂ClNO₃ | 1.00 |
| p-aminobenzoic acid | C₇H₇NO₂ | 0.20 |

| | | |
|---|---|---|
| ^{a}Trace elements solution ^{b}Vitamin solution | | |

Subsequently, 40ml of the content of the shake-flask was transferred into a fermenter (starting volume 0.4 L), which contained the medium as set out in Table 13.

**Table 13. Composition fermentation medium**

| **Raw material** | | **Final Concentration (g/kg)** |
|---|---|---|
| Glucose.1aq | C₆H₁₂O₆.1 H₂O | 66 |
| Ammonium sulphate | (NH₄)₂SO₄ | 1 |
| Potassium dihydrogen phosphate | KH₂PO₄ | 20 |
| Magnesium sulphate | MgSO₄. 7H₂O | 10 |
| Trace element solution | - | 16 |
| Vitamin solution | - | 16 |

The pH was controlled at 5.0 by addition of ammonia (10 wt%). Temperature was controlled at 30°C. pO₂ was controlled at 20% by adjusting the stirrer speed. Glucose concentration was kept limited by a controlled 60% glucose feed to the fermenter. RebM is determined as se out above in Example 6. The amount of RebM measured in the whole broth is as set out in Table 14.

**Table 14. RebM in whole broth**

| Strain | Time [h] | RebM [g/l] Whole Broth |
|---|---|---|
| STV2019 | 121 | 0.74 |

### Example 8 . Enzymatic conversion of glycosylated diterpenes into rebM

### Strain

*Saccharomyces cerevisiae* strain STV027 and STV2019 (see Examples 6 and 7 above) are cultivated on suitable media enabling active transcription and translation of the introduced genes. Obtained cells are pelleted and stored at -20 until analysis.

### Preparation Cell Free Extract

To 8 gr cell pellet 40mL of 100mM Tris buffer pH 7.18 is added and homogenized. Subsequently 8 gr glass beads (50-200um) are added. The samples were cooled on ice for 15 minutes. Cells are disrupted by 4 cycles of each 2 min vortexing on full speed followed by 5 min cooling on ice. After lysis the extract is centrifuged at 3000g for 60 min at 4 degrees C. The obtained supernatant is used directly for activity assays.

### Preparation Permeabilized Cells

8 gr of fresh pellet is homogenized with 40mL of 40% DMSO in Tris buffer (100mM pH 7.18) and frozen at -20 degrees C. Before analysis cells are thawed and 5mL is transferred to a new tube and washed three times with 100mM Tris buffer pH 7.18 containing 0.1% glucose. Cells were spun each time via centrifugation at 3000g. Finally, the cells are resuspended in 5mL of of 100mM Tris pH 7.18.

### UDP-Glucosyltransferases enzyme assay:

-2.5-500uL enzyme sample (CFE, permeabilized cells or isolated enzyme) is added to the reaction mixture containing:
- Glucose 0.05% (W/V; 5.55mM)
- UDP-Glc, 5mM
- MnCl₂, 3 mM
- DMSO, 2.5%(V/V)
- Steviol, RebaudiosideA or RebaudiosideD, 0.3 mM
- H₂O

The total reaction volume is 1000uL and the assay is performed in microtiterplates (MTP) heated to 30 degrees C in a MTP eppendorf incubator. Incubations are done while shaking at 250 rpm up to 120 hrs. The MTP's are sealed to prevent contamination and evaporation.

### Analysis

100uL samples were taken in time to follow the conversion reaction. Reaction mixtures are centrifuged at 4 degrees C for 20 minutes to stop the enzyme reaction and collect the samples. 50uL of acetonitrile is added to 100uL of sample in order to completely stop the reaction and extract all molecules formed. To this end the MTP's are sealed and shaken vigorously. Subsequently, the samples are centrifuged for 60 minutes at 4 degrees C and 100ul is transferred to a new MTP for LC-MS analysis (as described in Example 6, section 6.2).

### Results

Rebaudioside M is readily formed after 24 hrs (>1% of substrates converted) and steadily increases in concentration during the reaction for 120 hrs (>10% of substrates converted).

**Table 1: Description of the sequence listing**

| **Nucleic acid (CpO for *S. cerevisiae*)** | **Nucleic acid (CpO for *Y. lipolytica*)** | **Amino acid** | **Id*** | **UniProt^** | **Organism** |
|---|---|---|---|---|---|
| SEQ ID NO: 1 | SEQ ID NO: 151 | SEQ ID NO: 2 | CPS_1 | Q9FXV9 | Lactuca sativa (Garden Lettuce) |
| SEQ ID NO: 3 | SEQ ID NO: 152 | SEQ ID NO: 4 | tCPS_1 | | Lactuca sativa (Garden Lettuce) |
| SEQ ID NO: 5 | SEQ ID NO: 153 | SEQ ID NO: 6 | CPS_2 | D2X8G0 | Picea glauca |
| SEQ ID NO: 7 | SEQ ID NO: 154 | SEQ ID NO: 8 | CPS_3 | Q45221 | Bradyrhizobium japonicum |
| SEQ ID NO: 9 | SEQ ID NO: 155 | SEQ ID NO: 10 | KS_1 | Q9FXV8 | Lactuca sativa (Garden Lettuce) |
| SEQ ID NO: 11 | SEQ ID NO: 156 | SEQ ID NO: 12 | tKS_1 | | Lactuca sativa (Garden Lettuce) |
| SEQ ID NO: 13 | SEQ ID NO: 157 | SEQ ID NO: 14 | KS_2 | D2X8G1 | Picea glauca |
| SEQ ID NO: 15 | SEQ ID NO: 158 | SEQ ID NO: 16 | KS_3 | Q45222 | Bradyrhizobium japonicum |
| SEQ ID NO: 17 | SEQ ID NO: 159 | SEQ ID NO: 18 | CPSKS_1 | 013284 | Phaeosphaeria sp |
| SEQ ID NO: 19 | SEQ ID NO: 160 | SEQ ID NO: 20 | CPSKS_2 | Q9UVY5 | Gibberella fujikuroi |
| SEQ ID NO: 21 | SEQ ID NO: 161 | SEQ ID NO: 22 | KO_1 | B5MEX5 | Lactuca sativa (Garden Lettuce) |
| SEQ ID NO: 23 | SEQ ID NO: 162 | SEQ ID NO: 24 | KO_2 | B5MEX6 | Lactuca sativa (Garden Lettuce) |
| SEQ ID NO: 25 | SEQ ID NO: 163 | SEQ ID NO: 26 | KO_3 | B5DBY4 | Sphaceloma manihoticola |
| SEQ ID NO: 27 | SEQ ID NO: 164 | SEQ ID NO: 28 | KAH_1 | Q2HYU7 | Artemisia annua (Sweet wormwood). |
| SEQ ID NO: 29 | SEQ ID NO: 165 | SEQ ID NO: 30 | KAH_2 | B9SBP0 | Ricinus communis (Castor bean). |
| SEQ ID NO: 31 | SEQ ID NO: 166 | SEQ ID NO: 32 | **KAH_3** | Q0NZP1 | Stevia rebaudiana |
| SEQ ID NO: 33 | SEQ ID NO: 167 | SEQ ID NO: 34 | **KAH_4** | JP20090658 86 | Arabidopsis thaliana (Mouse-ear cress) |
| SEQ ID NO: 35 | SEQ ID NO: 168 | SEQ ID NO: 36 | UGT1_1 | A9X3L6 | Ixeris dentata var. albiflora. |
| SEQ ID NO: 37 | SEQ ID NO: 169 | SEQ ID NO: 38 | UGT1_2 | B9SIN2 | Ricinus communis (Castor bean). |
| SEQ ID NO: 39 | SEQ ID NO: 170 | SEQ ID NO: 40 | UGT3_1 | A9X3L7 | Ixeris dentata var. Albiflora |
| SEQ ID NO: 41 | SEQ ID NO: 171 | SEQ ID NO: 42 | UGT3_2 | B9IEM5 | Populus trichocarpa (Western balsam poplar) |
| SEQ ID NO: 43 | SEQ ID NO: 172 | SEQ ID NO: 44 | UGT3_3 | Q9M6E7 | Nicotiana tabacum |
| SEQ ID NO: 45 | SEQ ID NO: 173 | SEQ ID NO: 46 | UGT3_4 | A3E7Y9 | Vaccaria hispanica |
| SEQ ID NO: 47 | SEQ ID NO: 174 | SEQ ID NO: 48 | UGT3_5 | P10249 | Streptococcus mutans |
| SEQ ID NO: 49 | SEQ ID NO: 175 | SEQ ID NO: 50 | UGT4_1 | A4F1T4 | Lobelia erinus (Edging lobelia) |
| SEQ ID NO: 51 | SEQ ID NO: 176 | SEQ ID NO: 52 | UGT4_2 | Q9M052 | Arabidopsis thaliana (Mouse-ear cress) |
| SEQ ID NO: 53 | SEQ ID NO: 177 | SEQ ID NO: 54 | CPR_1 | Q7Z8R1 | Gibberella fujikuroi |
| SEQ ID NO: 55 | SEQ ID NO: 178 | SEQ ID NO: 56 | CPR_2 | Q9SB48 | Arabidopsis thaliana (Mouse-ear cress) |
| SEQ ID NO: 57 | SEQ ID NO: 179 | SEQ ID NO: 58 | CPR_3 | Q9SUM3 | Arabidopsis thaliana (Mouse-ear cress) |
| SEQ ID NO: 59 | SEQ ID NO: 141 | SEQ ID NO: 60 | CPS_SR | O22667 | Stevia rebaudiana |
| SEQ ID NO: 61 | SEQ ID NO: 142 | SEQ ID NO: 62 | tCPS_SR | | Stevia rebaudiana |
| SEQ ID NO: 63 | SEQ ID NO: 143 | SEQ ID NO: 64 | KS_SR | Q9XEI0 | Stevia rebaudiana |
| SEQ ID NO: 65 | SEQ ID NO: 144 | SEQ ID NO: 66 | tKS_SR | | Stevia rebaudiana |
| SEQ ID NO: 67 | SEQ ID NO: 145 | SEQ ID NO: 68 | KO_SR | Q4VCL5 | Stevia rebaudiana |
| SEQ ID NO: 69 | SEQ ID NO: 146 | SEQ ID NO: 70 | **KAH_SR** | US7927851 | Stevia rebaudiana |
| SEQ ID NO: 71 | SEQ ID NO: 147 | SEQ ID NO: 72 | UGT1_SR | Q6VAB0 | Stevia rebaudiana |
| SEQ ID NO: 73 | SEQ ID NO: 148 | SEQ ID NO: 74 | UGT3_SR | Q6VAA6 | Stevia rebaudiana |
| SEQ ID NO: 75 | SEQ ID NO: 149 | SEQ ID NO: 76 | UGT4_SR | Q6VAB4 | Stevia rebaudiana |
| SEQ ID NO: 77 | SEQ ID NO: 150 | SEQ ID NO: 78 | CPR_SR | Q2I6J8 | Stevia rebaudiana |
| SEQ ID NO: 79 | | SEQ ID NO: 80 | tHMG1 | G2WJY0 | Saccharomyces cerevisiae |
| SEQ ID NO: 81 | | SEQ ID NO: 82 | ERG20 | E7LW73 | Saccharomyces cerevisiae |
| SEQ ID NO: 83 | | SEQ ID NO: 84 | BTS1 | E7Q9V5 | Saccharomyces cerevisiae |
| SEQ ID NO: 85 | SEQ ID NO: 180 | SEQ ID NO: 86 | KO_Gibfu | 094142 | Gibberella fujikuroi |
| SEQ ID NO: 87 | SEQ ID NO: 181 | SEQ ID NO: 88 | UGT2_1a | B3VI56/99% | Stevia rebaudiana |
| SEQ iD NO: 89 | | SEQ ID NO: 90 | KAH_ASR1 | Xxx | S. rebaudiana |
| SEQ ID NO: 91 | | SEQ ID NO: 92 | KAH_ASR2 | Q0NZP1_STE RE | S. rebaudiana |
| SEQ ID NO: 93 | | SEQ ID NO: 94 | KAH_AAT | Q6NKZ8_AR ATH | A. thaliana |
| SEQ ID NO: 95 | | SEQ ID NO: 96 | KAH_AVV | F6H1G0_VIT VI//98% | Vitis vinifera |
| SEQ ID NO: 97 | | SEQ ID NO: 98 | KAH_AMT | Q2MJ20_ME DTR | Medicago truncatula |
| SEQ ID NO: 99 | | SEQ ID NO: 100 | UGT2_1b | B3VI56/99% | S. rebaudiana |
| SEQ ID NO: 101 | | SEQ ID NO: 102 | UGT2_2 | Q53UH5_IPO PU | I. purpurea |
| SEQ ID NO: 103 | | SEQ ID NO: 104 | UGT2_3 | UGAT_BELPE /99% | Bellis perennis |
| SEQ ID NO: 105 | | SEQ ID NO: 106 | UGT2_4 | B3VI56 | S. rebaudiana |
| SEQ iD NO: 107 | | SEQ ID NO: 108 | UGT2_5 | Q6VAA8 | S. rebaudiana |
| SEQ ID NO: 109 | | SEQ ID NO: 110 | UGT2_6 | Q8LKG3 | S. rebaudiana |
| SEQ ID NO: 111 | | SEQ ID NO: 112 | UGT2_7 | B9HSH7_PO PTR | Populus trichocarpa |
| SEQ ID NO: 113 | | SEQ ID NO: 114 | UGT_RD1 | Q6VAA3 | S. rebaudiana |
| SEQ ID NO: 115 | | SEQ ID NO: 116 | UGT_RD2 | Q8H6A4 | S. rebaudiana |
| SEQ ID NO: 117 | | SEQ ID NO: 118 | UGT_RD3 | Q6VAA4 | S. rebaudiana |
| SEQ ID NO: 119 | | SEQ ID NO: 120 | UGT_RD4 | Q6VAA5 | S. rebaudiana |
| SEQ ID NO: 121 | | SEQ ID NO: 122 | UGT_RD5 | Q6VAA7 | S. rebaudiana |
| SEQ ID NO: 123 | | SEQ ID NO: 124 | UGT_RD6 | Q6VAA8 | S. rebaudiana |
| SEQ ID NO: 125 | | SEQ ID NO: 126 | UGT_RD7 | Q6VAA9 | S. rebaudiana |
| SEQ ID NO: 127 | | SEQ ID NO: 128 | UGT_RD8 | Q6VAB1 | S. rebaudiana |
| SEQ ID NO: 129 | | SEQ ID NO: 130 | UGT_RD9 | Q6VAB2 | S. rebaudiana |
| SEQ ID NO: 131 | | SEQ ID NO: 132 | UGT_RD10 | Q6VAB3 | S. rebaudiana |
| SEQ ID NO: 133 | | SEQ ID NO: 134 | UGT_RD11 | B9VVB1 | S. rebaudiana |
| SEQ ID NO: 135 | | SEQ ID NO: 136 | UGT_RD12 | C7EA09 | S. rebaudiana |
| SEQ ID NO: 137 | | SEQ ID NO: 138 | UGT_RD13 | Q8LKG3 | S. rebaudiana |
| SEQ ID NO: 139 | | SEQ ID NO: 140 | UGT_RD14 | B3VI56 | S. rebaudiana |
| | SEQ ID NO: 182 | | tCPS | | |
| | SEQ ID NO: 183 | | tKS | | |
| | SEQ ID NO: 184 | | CPSKS | | |
| | SEQ ID NO: 185 | | KAH4 | | |
| | SEQ ID NO: 186 | | KO_Gibfu | | |
| | SEQ ID NO: 187 | | CPR1 | | |
| | SEQ ID NO: 188 | | CPR3 | | |
| | SEQ ID NO: 189 | | UGT1 | | |
| | SEQ ID NO: 190 | | UGT3 | | |
| | SEQ ID NO: 191 | | UGT4 | | |
| | SEQ ID NO: 192 | | UGT2_1a | | |
| | SEQ ID NO: 193 | | pTPI | | |
| | SEQ ID NO: 194 | | gpdT-pGPD | | |
| | SEQ ID NO: 195 | | pgmT-pTEF | | |
| | SEQ ID NO: 196 | | pgkT-pPGM | | |
| | SEQ ID NO: 197 | | LEU2 and flanking sequences | | |
| | SEQ ID NO: 198 | | vector sequences | | |
| | SEQ ID NO: 199 | | pENO | | |
| | SEQ ID NO: 200 | | HPH | | |
| SEQ ID NO: 201 | | | Sc Eno2.pro | | |
| SEQ ID NO: 202 | | | Sc Fbal.pro | | |
| SEQ ID NO: 203 | | | Sc Tefl.pro | | |
| SEQ ID NO: 204 | | | Sc Pgkl.pro | | |
| SEQ ID NO: 205 | | | Kl prom 12.pro | | |
| SEQ ID NO: 206 | | | Ag lox TEFl.pro | | |
| SEQ ID NO: 207 | | | Kl prom 6.pro | | |
| SEQ ID NO: 208 | | | Sc Pmal.pro | | |
| SEQ ID NO: 209 | | | Sc Vps68.pro | | |
| SEQ ID NO: 210 | | | Sc Oye2.pro | | |
| SEQ ID NO: 211 | | | KANMX ORF | | |
| SEQ ID NO: 212 | | | Adhl.ter | | |
| SEQ ID NO: 213 | | | Adh2.ter | | |
| SEQ ID NO: 214 | | | Gmpl.ter | | |
| SEQ ID NO: 215 | | | Sc Tall.ter | | |
| SEQ ID NO: 216 | | | Sc Tpil.ter | | |
| SEQ ID NO: 217 | | | Ag Tef1_lox.ter | | |
| SEQ ID NO: 218 | | | Sc Pdcl.ter | | |
| SEQ ID NO: 219 | | | Sc Tdhl.ter | | |
| SEQ ID NO: 220 | | | Sc Enol.ter | | |
| SEQ ID NO: 221 | | | Kl prom3.pro | | |
| SEQ ID NO: 222 | | | Kl prom2.pro | | |
| SEQ ID NO: 223 | | | Sc PRE3. Pro | | |
| | SEQ ID NO: 224 | | Yl_GSY1_3'_con_5_F w | | |
| | SEQ ID NO: 225 | | Yl_GSY1_3'_con_a_R v | | |
| | SEQ ID NO: 226 | | Yl_GSY1_5'_con_f_F w | | |
| | SEQ ID NO: 227 | | Yl_GSY1_5'_con_3_R v | | |
| | SEQ ID NO: 228 | | Con 5 fw | | |
| | SEQ ID NO: 229 | | Split KanMX rv | | |
| | SEQ ID NO: 230 | | Split KanMX fw | | |
| | SEQ ID NO: 231 | | Con 3 rv | | |
| | SEQ ID NO: 232 | | Lox66 | | |
| | SEQ ID NO: 233 | | Lox71 | | |
| | SEQ ID NO: 234 | | tHMGopt | | |
| | SEQ ID NO: 235 | | GGSopt | | |
| | SEQ ID NO: 236 | | tCPS_SR | | |
| | SEQ ID NO: 237 | | tKS_SR | | |
| | SEQ ID NO: 238 | | KO_Gib | | |
| | SEQ ID NO: 239 | | KAH_4 | | |
| | SEQ ID NO: 240 | | CPR_3 | | |
| | SEQ ID NO: 241 | | UGT1 | | |
| | SEQ ID NO: 242 | | UGT2 | | |
| | SEQ ID NO: 243 | | UGT3 | | |
| | SEQ ID NO: 244 | | UGT4 | | |
| | SEQ ID NO: 245 | | HPH | | |
| | SEQ ID NO: 246 | | NAT | | |
| | SEQ ID NO: 247 | | KAN | | |
| | SEQ ID NO: 248 | | KAN neoR | | |
| | SEQ ID NO: 249 | | CRE | | |
| | SEQ ID NO: 250 | | LEU2 | | |
| | SEQ ID NO: 251 | | URA2 blaster | | |
| | SEQ ID NO: 252 | | URA3 blaster | | |
| | SEQ ID NO: 253 | | pHSP | | |
| | SEQ ID NO: 254 | | pHYPO | | |
| | SEQ ID NO: 255 | | pENO | | |
| | SEQ ID NO: 256 | | pTPI | | |
| | SEQ ID NO: 257 | | pCWP | | |
| | SEQ ID NO: 258 | | pPGM | | |
| | SEQ ID NO: 259 | | YP005 | | |
| | SEQ ID NO: 260 | | SCP2 | | |
| | SEQ ID NO: 261 | | pTEF1 | | |
| | SEQ ID NO: 262 | | pHHF | | |
| | SEQ ID NO: 263 | | A.g. pTEF1 | | |
| | SEQ ID NO: 264 | | Ag_lox_TEF1 | | |
| | SEQ ID NO: 265 | | cwpT | | |
| | SEQ ID NO: 266 | | gpdT | | |
| | SEQ ID NO: 267 | | pgmT | | |
| | SEQ ID NO: 268 | | pgkT | | |
| | SEQ ID NO: 269 | | xprT | | |
| | SEQ ID NO: 270 | | hhfT | | |
| | SEQ ID NO: 271 | | A.g.tef1T | | |
| | SEQ ID NO: 272 | | gpdT | | |
| | SEQ ID NO: 273 | | pgmT | | |
| | SEQ ID NO: 274 | | pgkT | | |
| | SEQ ID NO: | 275 | Ag_tef1T-lox | | |

greyed out ids are truncated and thus a fragment of mentioned UniProt id

## Claims

1. A process for the preparation of rebaudioside M which comprises fermenting a microorganism belonging to the genus *Yarrowia* in a suitable fermentation medium at a pH of below 6, and optionally recovering the diterpene or glycosylated diterpene, wherein the microorganism is a recombinant microorganism comprising nucleotide sequence(s) encoding:
a polypeptide having ent-copalyl pyrophosphate synthase activity;
a polypeptide having ent-Kaurene synthase activity;
a polypeptide having ent-Kaurene oxidase activity; and
a polypeptide having kaurenoic acid 13-hydroxylase activity,
a polypeptide capable of catalyzing the addition of a glucose at the C-13 position of steviol to yield 13-*O*-steviolmonoside,
a polypeptide capable of catalyzing the addition of a glucose at the C-13 position of 13-*O*-steviolmonoside to yield steviolbioside or at the C-19 position of rebaudioside A to yield rebaudioside D,
a polypeptide capable of catalyzing the addition of a glucose at the C-19 position of steviolbioside to yield stevioside; and
a polypeptide capable of catalyzing addition of a glucose at the C-13 position of stevioside to yield rebaudioside A or at the C-19 position of rebaudioside D to yield rebaudioside M,
whereby expression of the nucleotide sequence(s) confer(s) on the microorganism the ability to produce at least rebaudioside M,
wherein the microorganism is capable of expressing a nucleotide sequence encoding a polypeptide having NADPH-cytochrome p450 reductase activity,
wherein the microorganism is capable of expressing:
a. a nucleotide sequence encoding a polypeptide having ent-copalyl pyrophosphate synthase activity, wherein said nucleotide sequence comprises:
i. a nucleotide sequence encoding a polypeptide having ent-copalyl pyrophosphate synthase activity, said polypeptide comprising an amino acid sequence that has at least 20% sequence identity with the amino acid sequence of SEQ ID NOs: 2, 4, 6, 8, 18, 20, 60 or 62;
ii. a nucleotide sequence that has at least 15% sequence identity with the nucleotide sequence of SEQ ID NOs: 1, 3, 5, 7, 17, 19, 59, 61, 141, 142, 151, 152, 153, 154, 159, 160, 182 or 184;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code,
b. a nucleotide sequence encoding a polypeptide having ent-Kaurene synthase activity, wherein said nucleotide sequence comprises:
i. a nucleotide sequence encoding a polypeptide having ent-Kaurene synthase activity, said polypeptide comprising an amino acid sequence that has at least 20% sequence identity with the amino acid sequence of SEQ ID NOs: 10, 12, 14, 16, 18, 20, 64 or 66;
ii. a nucleotide sequence that has at least 15% sequence identity with the nucleotide sequence of SEQ ID NOs: 9, 11, 13, 15, 17, 19, 63, 65, 143, 144, 155, 156, 157, 158, 159, 160, 183 or 184;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code,
c. a nucleotide sequence encoding a polypeptide having ent-Kaurene oxidase activity, wherein said nucleotide sequence comprises:
i. a nucleotide sequence encoding a polypeptide having ent-Kaurene oxidase activity, said polypeptide comprising an amino acid sequence that has at least 20% sequence identity with the amino acid sequence of SEQ ID NOs: 22, 24, 26, 68 or 86;
ii. a nucleotide sequence that has at least 15% sequence identity with the nucleotide sequence of SEQ ID NOs: 21, 23, 25, 67, 85, 145, 161, 162, 163, 180 or 186;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code; or
d. a nucleotide sequence encoding a polypeptide having kaurenoic acid 13-hydroxylase activity, wherein said nucleotide sequence comprises:
i. a nucleotide sequence encoding a polypeptide having kaurenoic acid 13-hydroxylase activity, said polypeptide comprising an amino acid sequence that has at least 20% sequence identity with the amino acid sequence of SEQ ID NOs: 28, 30, 32, 34, 70, 90, 92, 94, 96 or 98;
ii. a nucleotide sequence that has at least 15% sequence identity with the nucleotide sequence of SEQ ID NOs: 27, 29, 31, 33, 69, 89, 91, 93, 95, 97, 146, 164, 165, 166, 167 or 185;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

2. A process according to claim 1 for the preparation of a diterpene or glycosylated diterpene, wherein the process is carried out on an industrial scale.

3. A process according to any one of the preceding claims wherein the recombinant microorganism is capable of expressing a nucleotide sequence encoding a polypeptide capable of catalyzing the addition of a glucose at the C-13 position of steviol, wherein said nucleotide comprises:
i. a nucleotide sequence encoding a polypeptide capable of catalyzing the addition of a glucose at the C-13 position of steviol, said polypeptide comprising an amino acid sequence that has at least 20% sequence identity with the amino acid sequence of SEQ ID NOs: 36, 38 or 72;
ii. a nucleotide sequence that has at least 15% sequence identity with the nucleotide sequence of SEQ ID NOs: 35, 37, 71, 147, 168, 169, 189;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

4. A process according to any one of the preceding claims wherein the recombinant microorganism is capable of expressing a nucleotide sequence encoding a polypeptide capable of catalyzing the addition of a glucose at the C-13 position of steviolmonoside or at the C-19 position of rebaudioside A, wherein said nucleotide comprises:
i. a nucleotide sequence encoding a polypeptide capable of catalyzing the addition of a glucose at the C-13 position of steviolmonoside, said polypeptide comprising an amino acid sequence that has at least 20% sequence identity with the amino acid sequence of SEQ ID NOs: 88, 100, 102, 104, 106, 108, 110, 112;
ii. a nucleotide sequence that has at least 15% sequence identity with the nucleotide sequence of SEQ ID NOs: 87, 99, 101, 103, 105, 107, 109, 111, 181 or 192;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

5. A process according to any one of the preceding claims wherein the recombinant microorganism is capable of expressing a nucleotide sequence encoding a a polypeptide capable of catalyzing the addition of a glucose at the C-19 position of steviolbioside, wherein said nucleotide sequence comprises:
i. a nucleotide sequence encoding a polypeptide capable of catalyzing the addition of a glucose at the C-19 position of steviolbioside, said polypeptide comprising an amino acid sequence that has at least 20% sequence identity with the amino acid sequence of SEQ ID NOs: 40, 42, 44, 46, 48 or 74;
ii. a nucleotide sequence that has at least 15% sequence identity with the nucleotide sequence of SEQ ID NOs: 39, 41, 43, 45, 47, 73, 148, 170, 171, 172, 173, 174 or 190;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

6. A process according to any one of the preceding claims wherein the recombinant microorganism expresses a nucleotide sequence encoding a polypeptide capable of catalyzing addition of a glucose at the C-13 position of stevioside or at the C-19 position of rebaudioside D, wherein said nucleotide sequence comprises:
i. a nucleotide sequence encoding a polypeptide capable of catalyzing glucosylation of the C-3' of the glucose at the C-13 position of stevioside, said polypeptide comprising an amino acid sequence that has at least 20% sequence identity with the amino acid sequence of SEQ ID NOs: 50, 52 or 76;
ii. a nucleotide sequence that has at least 15% sequence identity with the nucleotide sequence of SEQ ID NOs: 49, 51 or 75, 149, 175, 176 or 191;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

7. A process according to any one of the preceding claims wherein the recombinant microorganism is a *Yarrowia lipolytica* cell.

8. A process according to any one of the preceding claims wherein wherein the ability of the microorganism to produce geranylgeranyl diphosphate (GGPP) is upregulated.

9. A process according to any one of the preceding claims wherein the recombinant microorganism, comprises one or more nucleotide sequence(s) encoding hydroxymethylglutaryl-CoA reductase, farnesyl-pyrophosphate synthetase and geranylgeranyl diphosphate synthase, whereby expression of the nucleotide sequence(s) confer(s) on the microorganism the ability to produce elevated levels of GGPP.

10. A process according to any one of the preceding claims wherein the recombinant microorganism is capable of expressing one or more of:
a. a nucleotide sequence encoding a polypeptide having hydroxymethylglutaryl-CoA reductase activity, wherein said nucleotide sequence comprises:
i. a nucleotide sequence encoding a polypeptide having hydroxymethylglutaryl-CoA reductase activity, said polypeptide comprising an amino acid sequence that has at least 20% sequence identity with the amino acid sequence of SEQ ID NO: 80;
ii. a nucleotide sequence that has at least 15% sequence identity with the nucleotide sequence of SEQ ID NO: 79;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code,
b. a nucleotide sequence encoding a polypeptide having farnesyl-pyrophosphate synthetase activity, wherein said nucleotide sequence comprises:
i. a nucleotide sequence encoding a polypeptide having farnesyl-pyrophosphate synthetase activity, said polypeptide comprising an amino acid sequence that has at least 20% sequence identity with the amino acid sequence of SEQ ID NO: 82;
ii. a nucleotide sequence that has at least 15% sequence identity with the nucleotide sequence of SEQ ID NOs: 81;
iii. a nucleotide sequence the complementary strand of which hybridizes to a nucleic acid molecule of sequence of (i) or (ii); or
iv. a nucleotide sequence which differs from the sequence of a nucleic acid molecule of (iii) due to the degeneracy of the genetic code; or
c. a nucleotide sequence encoding a polypeptide having geranylgeranyl diphosphate synthase activity, wherein said nucleotide sequence comprises:
i. a nucleotide sequence encoding a polypeptide having geranylgeranyl diphosphate synthase activity, said polypeptide comprising an amino acid sequence that has at least 20% sequence identity with the amino acid sequence of SEQ ID NO: 84;
ii. a nucleotide sequence that has at least 15% sequence identity with the nucleotide sequence of SEQ ID NOs: 83;
iii. a nucleotide sequence the complementary strand of which hybridizes to a nucleic acid molecule of sequence of (i) or (ii); or
iv. a nucleotide sequence which differs from the sequence of a nucleic acid molecule of (i), (ii) or (iii) due to the degeneracy of the genetic code.

## Patentansprüche

1. Verfahren zur Herstellung von Rebaudiosid M, welches das Fermentieren eines Mikroorganismus der Gattung *Yarrowia* in einem geeigneten Fermentationsmedium bei einem pH-Wert von unter 6 und gegebenenfalls das Isolieren des Diterpens bzw. des glykosylierten Diterpens umfasst, wobei es sich bei dem Mikroorganismus um einen rekombinanten Mikroorganismus handelt, der eine oder mehrere Nukleotidsequenzen umfasst, die für Folgendes codieren:
ein Polypeptid mit ent-Copalylpyrophosphatsynthaseaktivität,
ein Polypeptid mit ent-Kaurensynthaseaktivität, ein Polypeptid mit ent-Kaurenoxidaseaktivität und
ein Polypeptid mit Kaurensäure-13-Hydroxylaseaktivität,
ein Polypeptid, das dazu fähig ist, die Addition von Glucose an der C-13-Position von Steviol unter Erhalt von 13-*O*-Steviolmonosid zu katalysieren,
ein Polypeptid, das dazu fähig ist, die Addition von Glucose an der C-13-Position von 13-*O-*Steviolmonosid unter Erhalt von Steviolbiosid oder an der C-19-Position von Rebaudiosid A unter Erhalt von Rebaudiosid D zu katalysieren,
ein Polypeptid, das dazu fähig ist, die Addition von Glucose an der C-19-Position von Steviolbiosid unter Erhalt von Steviosid zu katalysieren,
ein Polypeptid, das dazu fähig ist, die Addition von Glucose an der C-13-Position von 13-*O-*Steviosid unter Erhalt von Rebaudiosid A oder an der C-19-Position von Rebaudiosid D unter Erhalt von Rebaudiosid M zu katalysieren,
wobei die Expression der Nukleotidsequenz(en) dem Mikroorganismus die Fähigkeit zum Produzieren von mindestens Rebaudiosid M verleiht,
wobei der Mikroorganismus dazu fähig ist, eine Nukleotidsequenz zu exprimieren, die für ein Polypeptid mit NADPH-Cytochrom-p450-Reduktaseaktivität codiert,
wobei der Mikroorganismus dazu fähig ist, Folgendes zu exprimieren:
a. eine Nukleotidsequenz, die für ein Polypeptid mit ent-Copalylpyrophosphatsynthaseaktivität codiert, wobei die Nukleotidsequenz Folgendes umfasst:
i. eine Nukleotidsequenz, die für ein Polypeptid mit ent-Copalylpyrophosphatsynthaseaktivität codiert, wobei das Polypeptid eine Aminosäuresequenz umfasst, die mindestens 20% Sequenzidentität mit der Aminosäuresequenz von SEQ ID NOs: 2, 4, 6, 8, 18, 20, 60 oder 62 aufweist,
ii. eine Nukleotidsequenz, die mindestens 15% Sequenzidentität mit der Nukleotidsequenz von SEQ ID NOs: 1, 3, 5, 7, 17, 19, 59, 61, 141, 142, 151, 152, 153, 154, 159, 160, 182 oder 184 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet,
b. eine Nukleotidsequenz, die für ein Polypeptid mit ent-Kaurensynthaseaktivität codiert, wobei die Nukleotidsequenz Folgendes umfasst:
i. eine Nukleotidsequenz, die für ein Polypeptid mit ent-Kaurensynthaseaktivität codiert, wobei das Polypeptid eine Aminosäuresequenz umfasst, die mindestens 20% Sequenzidentität mit der Aminosäuresequenz von SEQ ID NOs: 10, 12, 14, 16, 18, 20, 64 oder 66 aufweist,
ii. eine Nukleotidsequenz, die mindestens 15% Sequenzidentität mit der Nukleotidsequenz von SEQ ID NOs: 9, 11, 13, 15, 17, 19, 63, 65, 143, 144, 155, 156, 157, 158, 159, 160, 183 oder 184 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet,
c. eine Nukleotidsequenz, die für ein Polypeptid mit ent-Kaurenoxidaseaktivität codiert, wobei die Nukleotidsequenz Folgendes umfasst:
i. eine Nukleotidsequenz, die für ein Polypeptid mit ent-Kaurenoxidaseaktivität codiert, wobei das Polypeptid eine Aminosäuresequenz umfasst, die mindestens 20% Sequenzidentität mit der Aminosäuresequenz von SEQ ID NOs: 22, 24, 26, 68 oder 86 aufweist,
ii. eine Nukleotidsequenz, die mindestens 15% Sequenzidentität mit der Nukleotidsequenz von SEQ ID NOs: 21, 23, 25, 67, 85, 145, 161, 162, 163, 180 oder 186 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet,
d. eine Nukleotidsequenz, die für ein Polypeptid mit Kaurensäure-13-Hydroxylaseaktivität codiert, wobei die Nukleotidsequenz Folgendes umfasst:
i. eine Nukleotidsequenz, die für ein Polypeptid mit Kaurensäure-13-Hydroxylaseaktivität codiert, wobei das Polypeptid eine Aminosäuresequenz umfasst, die mindestens 20% Sequenzidentität mit der Aminosäuresequenz von SEQ ID NOs: 28, 30, 32, 34, 70, 90, 92, 94, 96 oder 98 aufweist,
ii. eine Nukleotidsequenz, die mindestens 15% Sequenzidentität mit der Nukleotidsequenz von SEQ ID NOs: 27, 29, 31, 33, 69, 89, 91, 93, 95, 97, 146, 164, 165, 166, 167 oder 185 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

2. Verfahren nach Anspruch 1 zur Herstellung eines Diterpens oder glykosylierten Diterpens, wobei das Verfahren im großtechnischen Maßstab durchgeführt wird.

3. Verfahren nach einem der vorhergehenden Ansprüche, wobei der rekombinante Mikroorganismus dazu fähig ist, eine Nukleotidsequenz zu exprimieren, die für ein Polypeptid codiert, das die Addition einer Glucose in der C-13-Position von Steviol katalysieren kann, wobei das Nukleotid Folgendes umfasst:
i. eine Nukleotidsequenz, die für ein Polypeptid, das die Addition einer Glucose in der C-13-Position von Steviol katalysieren kann, codiert, wobei das Polypeptid eine Aminosäuresequenz umfasst, die mindestens 20% Sequenzidentität mit der Aminosäuresequenz von SEQ ID NOs: 36, 38 oder 72 aufweist,
ii. eine Nukleotidsequenz, die mindestens 15% Sequenzidentität mit der Nukleotidsequenz von SEQ ID NOs: 35, 37, 71, 147, 168, 169 oder 189 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

4. Verfahren nach einem der vorhergehenden Ansprüche, wobei der rekombinante Mikroorganismus dazu fähig ist, eine Nukleotidsequenz zu exprimieren, die für ein Polypeptid codiert, das die Addition einer Glucose in der C-13-Position von Steviolmonosid oder an der C-19-Position von Rabaudiosid A katalysieren kann, wobei das Nukleotid Folgendes umfasst:
i. eine Nukleotidsequenz, die für ein Polypeptid, das die Addition einer Glucose in der C-13-Position von Steviolmonosid katalysieren kann, codiert, wobei das Polypeptid eine Aminosäuresequenz umfasst, die mindestens 20% Sequenzidentität mit der Aminosäuresequenz von SEQ ID NOs: 88, 100, 102, 104, 106, 108, 110, 112 aufweist,
ii. eine Nukleotidsequenz, die mindestens 15% Sequenzidentität mit der Nukleotidsequenz von SEQ ID NOs: 87, 99, 101, 103, 105, 107, 109, 111, 181 oder 192 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

5. Verfahren nach einem der vorhergehenden Ansprüche, wobei der rekombinante Mikroorganismus dazu fähig ist, eine Nukleotidsequenz zu exprimieren, die für ein Polypeptid codiert, das die Addition einer Glucose in der C-19-Position von Steviolbiosid katalysieren kann, wobei die Nukleotidsequenz Folgendes umfasst:
i. eine Nukleotidsequenz, die für ein Polypeptid, das die Addition einer Glucose in der C-19-Position von Steviolbiosid katalysieren kann, codiert, wobei das Polypeptid eine Aminosäuresequenz umfasst, die mindestens 20% Sequenzidentität mit der Aminosäuresequenz von SEQ ID NOs: 40, 42, 44, 46, 48 oder 74 aufweist,
ii. eine Nukleotidsequenz, die mindestens 15% Sequenzidentität mit der Nukleotidsequenz von SEQ ID NOs: 39, 41, 43, 45, 47, 73, 148, 170, 171, 172, 173, 174 oder 190 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

6. Verfahren nach einem der vorhergehenden Ansprüche, wobei der rekombinante Mikroorganismus eine Nukleotidsequenz exprimiert, die für ein Polypeptid codiert, das die Addition einer Glucose an der C-13-Position von Steviosid oder an der C-19-Position von Rebaudiosid D katalysieren kann, wobei die Nukleotidsequenz Folgendes umfasst:
i. eine Nukleotidsequenz, die für ein Polypeptid codiert, das die Glucosylierung des C-3' der Glucose an die C-13-Position von Steviosid katalysieren kann, wobei das Polypeptid eine Aminosäuresequenz umfasst, die mindestens 20% Sequenzidentität mit der Aminosäuresequenz von SEQ ID NOs: 50, 52 oder 76 aufweist,
ii. eine Nukleotidsequenz, die mindestens 15% Sequenzidentität mit der Nukleotidsequenz von SEQ ID NOs: 49, 51, 75, 149, 175, 176 oder 191 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

7. Verfahren nach einem der vorhergehenden Ansprüche, wobei es sich bei dem rekombinanten Mikroorganismus um eine *Yarrowia lipolytica*-Zelle handelt.

8. Verfahren nach einem der vorhergehenden Ansprüche, wobei die Fähigkeit des Mikroorganismus zur Produktion von Geranylgeranyldiphosphat (GGPP) hochreguliert ist

9. Verfahren nach einem der vorhergehenden Ansprüche, wobei der rekombinante Mikroorganismus eine oder mehrere Nukleotidsequenzen umfasst, die für Hydroxymethylglutaryl-CoA-Reduktase, Farnesylpyrophosphatsynthetase und Geranylgeranyldiphosphatsynthase codieren, wobei die Expression der Nukleotidsequenz(en) dem Mikroorganismus die Fähigkeit zur Produktion erhöhter GGPP-Konzentrationen verleiht.

10. Verfahren nach einem der vorhergehenden Ansprüche, wobei der rekombinante Mikroorganismus dazu fähig ist, eine oder mehrere der Folgenden zu exprimieren:
a. eine Nukleotidsequenz, die für ein Polypeptid mit Hydroxymethylglutaryl-CoA-Reduktaseaktivität codiert, wobei die Nukleotidsequenz Folgendes umfasst:
i. eine Nukleotidsequenz, die für ein Polypeptid mit Hydroxymethylglutaryl-CoA-Reduktaseaktivität codiert, wobei das Polypeptid eine Aminosäuresequenz umfasst, die mindestens 20% Sequenzidentität mit der Aminosäuresequenz von SEQ ID NO: 80 aufweist,
ii. eine Nukleotidsequenz, die mindestens 15% Sequenzidentität mit der Nukleotidsequenz von SEQ ID NO: 79 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet,
b. eine Nukleotidsequenz, die für ein Polypeptid mit Farnesylpyrophosphatsynthetaseaktivität codiert, wobei die Nukleotidsequenz Folgendes umfasst:
i. eine Nukleotidsequenz, die für ein Polypeptid mit Farnesylpyrophosphatsynthetaseaktivität codiert, wobei das Polypeptid eine Aminosäuresequenz umfasst, die mindestens 20% Sequenzidentität mit der Aminosäuresequenz von SEQ ID NO: 82 aufweist,
ii. eine Nukleotidsequenz, die mindestens 15% Sequenzidentität mit der Nukleotidsequenz von SEQ ID NO: 81 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (iii) unterscheidet, oder
c. eine Nukleotidsequenz, die für ein Polypeptid mit Geranylgeranyldiphosphatsynthaseaktivität codiert, wobei die Nukleotidsequenz Folgendes umfasst:
i. eine Nukleotidsequenz, die für ein Polypeptid mit Geranylgeranyldiphosphatsynthaseaktivität codiert, wobei das Polypeptid eine Aminosäuresequenz umfasst, die mindestens 20% Sequenzidentität mit der Aminosäuresequenz von SEQ ID NO: 84 aufweist,
ii. eine Nukleotidsequenz, die mindestens 15% Sequenzidentität mit der Nukleotidsequenz von SEQ ID NO: 83 aufweist,
iii. eine Nukleotidsequenz, deren komplementärer Strang mit einem Nukleinsäuremolekül der Sequenz von (i) oder (ii) hybridisiert, oder
iv. eine Nukleotidsequenz, die sich aufgrund der Degeneration des genetischen Codes von der Sequenz eines Nukleinsäuremoleküls gemäß (i), (ii) oder (iii) unterscheidet.

## Revendications

1. Procédé de préparation de rébaudioside M qui comprend la fermentation d'un microorganisme appartenant au genre *Yarrowia* dans un milieu de fermentation adapté à un pH inférieur à 6, et facultativement la récupération du diterpène ou du diterpène glycosylé, dans lequel le microorganisme est un microorganisme recombinant comprenant une ou plusieurs séquences nucléotidiques codant pour :
un polypeptide ayant une activité ent-copalyle pyrophosphate synthase ;
un polypeptide ayant une activité ent-kaurène synthase ;
un polypeptide ayant une activité ent-kaurène oxydase ; et
un polypeptide ayant une activité acide kaurénoïque 13-hydroxylase,
un polypeptide capable de catalyser l'addition d'un glucose à la position C-13 du stéviol pour obtenir le 13-O-stéviolmonoside,
un polypeptide capable de catalyser l'addition d'un glucose à la position C-13 du 13-*O*-stéviolmonoside pour obtenir le stéviolbioside ou à la position C-19 du rébaudioside A pour obtenir le rébaudioside D,
un polypeptide capable de catalyser l'addition d'un glucose à la position C-19 du stéviolbioside pour obtenir le stévioside ; et
un polypeptide capable de catalyser l'addition d'un glucose à la position C-13 du stévioside pour obtenir le rébaudioside A ou à la position C-19 du rébaudioside D pour obtenir le rébaudioside M,
dans lequel l'expression des une ou plusieurs séquences nucléotidiques confère au microorganisme la capacité de produire au moins le rébaudioside M,
dans lequel le microorganisme est capable d'exprimer une séquence nucléotidique codant pour un polypeptide ayant une activité NADPH-cytochrome p450 réductase,
dans lequel le microorganisme est capable d'exprimer :
a. une séquence nucléotidique codant pour un polypeptide ayant une activité ent-copalyle pyrophosphate synthase, où ladite séquence nucléotidique comprend :
i. une séquence nucléotidique codant pour un polypeptide ayant une activité ent-copalyle pyrophosphate synthase, ledit polypeptide comprenant une séquence d'acides aminés qui présente au moins 20 % d'identité de séquence avec la séquence d'acides aminés de SEQ ID NO : 2, 4, 6, 8, 18, 20, 60 ou 62 ;
ii. une séquence nucléotidique qui présente au moins 15 % d'identité de séquence avec la séquence nucléotidique de SEQ ID NO : 1, 3, 5, 7, 17, 19, 59, 61, 141, 142, 151, 152, 153, 154, 159, 160, 182 ou 184 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique,
b. une séquence nucléotidique codant pour un polypeptide ayant une activité ent-kaurène synthase, où ladite séquence nucléotidique comprend :
i. une séquence nucléotidique codant pour un polypeptide ayant une activité ent-kaurène synthase, ledit polypeptide comprenant une séquence d'acides aminés qui présente au moins 20 % d'identité de séquence avec la séquence d'acides aminés de SEQ ID NO : 10, 12, 14, 16, 18, 20, 64 ou 66 ;
ii. une séquence nucléotidique qui présente au moins 15 % d'identité de séquence avec la séquence nucléotidique de SEQ ID NO : 9, 11, 13, 15, 17, 19, 63, 65, 143, 144, 155, 156, 157, 158, 159, 160, 183 ou 184 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique,
c. une séquence nucléotidique codant pour un polypeptide ayant une activité ent-kaurène oxydase, où ladite séquence nucléotidique comprend :
i. une séquence nucléotidique codant pour un polypeptide ayant une activité ent-kaurène oxydase, ledit polypeptide comprenant une séquence d'acides aminés qui présente au moins 20 % d'identité de séquence avec la séquence d'acides aminés de SEQ ID NO : 22, 24, 26, 68 ou 86 ;
ii. une séquence nucléotidique qui présente au moins 15 % d'identité de séquence avec la séquence nucléotidique de SEQ ID NO : 21, 23, 25, 67, 85, 145, 161, 162, 163, 180 ou 186 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique ; ou
d. une séquence nucléotidique codant pour un polypeptide ayant une activité acide kaurénoïque 13-hydroxylase, où ladite séquence nucléotidique comprend :
i. une séquence nucléotidique codant pour un polypeptide ayant une activité acide kaurénoïque 13-hydroxylase, ledit polypeptide comprenant une séquence d'acides aminés qui présente au moins 20 % d'identité de séquence avec la séquence d'acides aminés de SEQ ID NO : 28, 30, 32, 34, 70, 90, 92, 94, 96 ou 98 ;
ii. une séquence nucléotidique qui présente au moins 15 % d'identité de séquence avec la séquence nucléotidique de SEQ ID NO : 27, 29, 31, 33, 69, 89, 91, 93, 95, 97, 146, 164, 165, 166, 167 ou 185 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.

2. Procédé selon la revendication 1 pour la préparation d'un diterpène ou diterpène glycosylé, où le procédé est conduit à une échelle industrielle.

3. Procédé selon l'une quelconque des revendications précédentes dans lequel le microorganisme recombinant est capable d'exprimer une séquence nucléotidique codant pour un polypeptide capable de catalyser l'addition d'un glucose à la position C-13 du stéviol, dans lequel ledit nucléotide comprend :
i. une séquence nucléotidique codant pour un polypeptide capable de catalyser l'addition d'un glucose à la position C-13 du stéviol, ledit polypeptide comprenant une séquence d'acides aminés qui présente au moins 20 % d'identité de séquence avec la séquence d'acides aminés de SEQ ID NO : 36, 38 ou 72 ;
ii. une séquence nucléotidique qui présente au moins 15 % d'identité de séquence avec la séquence nucléotidique de SEQ ID NO : 35, 37, 71, 147, 168, 169, 189 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.

4. Procédé selon l'une quelconque des revendications précédentes dans lequel le microorganisme recombinant est capable d'exprimer une séquence nucléotidique codant pour un polypeptide capable de catalyser l'addition d'un glucose à la position C-13 du stéviolmonoside ou à la position C-19 du rébaudioside A, où ledit nucléotide comprend :
i. une séquence nucléotidique codant pour un polypeptide capable de catalyser l'addition d'un glucose à la position C-13 du stéviolmonoside, ledit polypeptide comprenant une séquence d'acides aminés qui présente au moins 20 % d'identité de séquence avec la séquence d'acides aminés de SEQ ID NO : 88, 100, 102, 104, 106, 108, 110, 112 ;
ii. une séquence nucléotidique qui présente au moins 15 % d'identité de séquence avec la séquence nucléotidique de SEQ ID NO : 87, 99, 101, 103, 105, 107, 109, 111, 181 ou 192 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.

5. Procédé selon l'une quelconque des revendications précédentes dans lequel le microorganisme recombinant est capable d'exprimer une séquence nucléotidique codant pour un polypeptide capable de catalyser l'addition d'un glucose à la position C-19 du stéviolbioside, où ladite séquence nucléotidique comprend :
i. une séquence nucléotidique codant pour un polypeptide capable de catalyser l'addition d'un glucose à la position C-19 du stéviolbioside, ledit polypeptide comprenant une séquence d'acides aminés qui présente au moins 20 % d'identité de séquence avec la séquence d'acides aminés de SEQ ID NO : 40, 42, 44, 46, 48 ou 74 ;
ii. une séquence nucléotidique qui présente au moins 15 % d'identité de séquence avec la séquence nucléotidique de SEQ ID NO : 39, 41, 43, 45, 47, 73, 148, 170, 171, 172, 173, 174 ou 190 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.

6. Procédé selon l'une quelconque des revendications précédentes dans lequel le microorganisme recombinant exprime une séquence nucléotidique codant pour un polypeptide capable de catalyser l'addition d'un glucose à la position C-13 du stévioside ou à la position C-19 du rébaudioside D, où ladite séquence nucléotidique comprend :
i. une séquence nucléotidique codant pour un polypeptide capable de catalyser la glucosylation du C-3' du glucose à la position C-13 du stévioside, ledit polypeptide comprenant une séquence d'acides aminés qui présente au moins 20 % d'identité de séquence avec la séquence d'acides aminés de SEQ ID NO : 50, 52 ou 76 ;
ii. une séquence nucléotidique qui présente au moins 15 % d'identité de séquence avec la séquence nucléotidique de SEQ ID NO : 49, 51 ou 75, 149, 175, 176 ou 191 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.

7. Procédé selon l'une quelconque des revendications précédentes dans lequel le microorganisme recombinant est une cellule de *Yarrowia lipolytica.*

8. Procédé selon l'une quelconque des revendications précédentes dans lequel la capacité du microorganisme à produire le géranylgéranyle diphosphate (GGPP) est régulée positivement.

9. Procédé selon l'une quelconque des revendications précédentes dans lequel le microorganisme recombinant comprend une ou plusieurs séquences nucléotidiques codant pour l'hydroxyméthylglutaryl-CoA réductase, la farnésyl-pyrophosphate synthétase et la géranylgéranyle diphosphate synthase, dans lequel l'expression des une ou plusieurs séquences nucléotidiques confère au microorganisme la capacité de produire des taux élevés de GGPP.

10. Procédé selon l'une quelconque des revendications précédentes dans lequel le microorganisme recombinant est capable d'exprimer l'une ou plusieurs de :
a. une séquence nucléotidique codant pour un polypeptide ayant une activité hydroxyméthylglutaryl-CoA réductase, où ladite séquence nucléotidique comprend :
i. une séquence nucléotidique codant pour un polypeptide ayant une activité hydroxyméthylglutaryl-CoA réductase, ledit polypeptide comprenant une séquence d'acides aminés qui présente au moins 20 % d'identité de séquence avec la séquence d'acides aminés de SEQ ID NO : 80 ;
ii. une séquence nucléotidique qui présente au moins 15 % d'identité de séquence avec la séquence nucléotidique de SEQ ID NO : 79 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique,
b. une séquence nucléotidique codant pour un polypeptide ayant une activité farnésyl-pyrophosphate synthétase, où ladite séquence nucléotidique comprend :
i. une séquence nucléotidique codant pour un polypeptide ayant une activité farnésyl-pyrophosphate synthétase, ledit polypeptide comprenant une séquence d'acides aminés qui présente au moins 20 % d'identité de séquence avec la séquence d'acides aminés de SEQ ID NO : 82 ;
ii. une séquence nucléotidique qui présente au moins 15 % d'identité de séquence avec la séquence nucléotidique de SEQ ID NO : 81 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (iii) en raison de la dégénérescence du code génétique ; ou
c. une séquence nucléotidique codant pour un polypeptide ayant une activité géranylgéranyle diphosphate synthase, où ladite séquence nucléotidique comprend :
i. une séquence nucléotidique codant pour un polypeptide ayant une activité géranylgéranyle diphosphate synthase, ledit polypeptide comprenant une séquence d'acides aminés qui présente au moins 20 % d'identité de séquence avec la séquence d'acides aminés de SEQ ID NO : 84 ;
ii. une séquence nucléotidique qui présente au moins 15 % d'identité de séquence avec la séquence nucléotidique de SEQ ID NO : 83 ;
iii. une séquence nucléotidique dont le brin complémentaire s'hybride avec une molécule d'acide nucléique ayant la séquence de (i) ou (ii) ; ou
iv. une séquence nucléotidique qui diffère de la séquence d'une molécule d'acide nucléique de (i), (ii) ou (iii) en raison de la dégénérescence du code génétique.
